# FASCICULE DE BREVET EUROPEEN

(11) **EP 3 116 527 B1**
(45) Date de publication et mention de la délivrance du brevet: **04.09.2019**
(21) Numéro de dépôt: 15717016.8
(22) Date de dépôt: 10.03.2015
(51) Int. Cl.: A61K 38/17, A61K 38/36, A61K 38/38, A61K 39/395, C07K 1/22

(54) **PROCÉDÉ DE PRÉPARATION DE PROTÉINES PLASMATIQUES HUMAINES**
VERFAHREN ZUR HERSTELLUNG VON MENSCHLICHEN PLASMAPROTEINEN
METHOD FOR PREPARING HUMAN PLASMA PROTEINS

(30) Priorité: 11.03.2014 FR 1451997
(43) Date de publication de la demande: 18.01.2017
(73) Titulaire: Laboratoire Français du Fractionnement et des Biotechnologies, 91940 Les Ulis (FR)
(72) Inventeur: BATAILLE, Damien, F-91540 Ormoy (FR); CHTOUROU, Abdessatar, F-78990 Elancourt (FR); SANTAMBIEN, Patrick, F-92120 Montrouge (FR)
(74) Mandataire: Cabinet Becker et Associés
(86) Numéro de dépôt international: PCT/FR2015/050598
(87) Numéro de publication internationale: WO 2015/136217

(56) Documents cités:
- EP-A1- 1 775 001
- WO-A1-99/64462
- WO-A1-2013/007740
- FR-A1- 2 824 568
- FR-A1- 2 895 263
- FR-A1- 2 942 233
- Lars Aumann ET AL: "A continuous multicolumn countercurrent solvent gradient purification (MCSGP) process", Biotechnology and bioengineering, 1 December 2007 (2007-12-01), pages 1043-1055, XP055458239, United States DOI: 10.1002/bit.21527 Retrieved from the Internet: URL:http://www.engconf.org/staging/wp-cont ent/uploads/2013/12/1-Morbidelli-131014_Pr esentation_Barcelona_MM.pdf [retrieved on 2017-03-12]

## Description

L'invention concerne un procédé de préparation de protéines plasmatiques humaines à usage thérapeutique, à partir de plasma sanguin ou d'une fraction plasmatique.

### Contexte de l'invention :

De nombreuses pathologies sont actuellement traitées par des fractions issues de plasma sanguin enrichies en une ou plusieurs protéines plasmatiques. Ainsi, les facteurs de coagulation sont généralement utilisés en thérapie substitutive, pour la prévention ou le traitement des hémorragies associées à des déficits en facteurs de coagulation. Le fibrinogène est le plus souvent prescrit pour le traitement des complications associées à l'afibrinogénémie constitutionnelle ou sévère et les syndromes hémorragiques ou risques d'hémorragies associés à une hypofibrinogénémie. L'albumine est quant à elle destinée à la restauration et au maintien du volume sanguin circulant (hypovolémie confirmée). De même, de nombreuses pathologies sont actuellement traitées par des fractions de plasma sanguin enrichies en immunoglobulines, et notamment en immunoglobulines G (IgG), comprenant généralement plus de 95% d'Ig.

Par exemple, des fractions de plasma sanguin enrichies en immunoglobulines G, ou concentrés d'IgG, sont utilisées pour corriger les déficits immunitaires primitifs avec défaut de production d'anticorps, certains déficits immunitaires secondaires, tels que les leucémies, myélomes ou infections récidivantes, etc. L'administration d'Ig peut également avoir un effet bénéfique dans le traitement du Purpura Thrombopénique Idiopathique (PTI) de l'enfant et de l'adulte, du syndrome de Guillain-Barré, des polyneuropathies démyélinisantes, de la neuropathie motrice multifocale, de la polyradiculonévrites inflammatoires démyélinisantes chroniques (PIDC), de la maladie de Kawasaki, de la sclérose en plaque, de la dermatomyosite cortico-résistante, de la myasthénie aiguë, de la rétinochoroïdite de Birdshot, de l'ictère néonatal par incompatibilité foeto-maternelle dans le système ABO (maladie hémolytique du nouveau-né par incompatibilité ABO) etc.

Les indications thérapeutiques multiples, ainsi que les doses très élevées qui peuvent dans certains cas être prescrites (pouvant aller jusqu'à 2 g d'immunoglobulines/kg/jour), ont engendré des situations de tensions extrêmes sur les approvisionnements, pouvant aller jusqu'à des pénuries en Europe et aux Etats-Unis d'Amérique.

Aussi, de nombreux procédés ont été développés pour la purification des protéines plasmatiques à partir de plasma sanguin. Le procédé industriel permettant l'extraction des protéines plasmatiques pour un usage thérapeutique est appelé « fractionnement plasmatique ». Les techniques de fractionnement utilisées de nos jours débutent majoritairement par une étape de cryoprécipitation, consistant en une décongélation du plasma à basse température pour isoler un cryoprécipité enrichi en facteur VIII, facteur de Willebrand, fibrinogène et fibronectine. Les protéines de la fraction surnageante (surnageant de cryoprécipité) peuvent ensuite être séparées par précipitations séquentielles en présence d'éthanol (Cohn et al. 1946, J. Am. Chem. Soc. 68, 459).

Plusieurs variantes du procédé original de Cohn utilisant également un fractionnement éthanolique ont été décrites. Ainsi, la publication de Tanaka K. et al., 2000 (Braz J Med Bio Res 2000, 33(1): 27-30) décrit un procédé de purification d'immunoglobulines G à partir de fractions " I+II+II " et " II+III " obtenues après fractionnement éthanolique selon la méthode de Cohn, par séparation par chromatographie sur trois types de gels, deux gels échangeurs d'ions (Q-Sepharose FF et CM-Sepharose FF) et une étape de gel filtration (Sephacryl S-300 HR).

Une voie alternative à la précipitation à l'éthanol a été décrite par Steinbuch et al. (Rev. Franc. Et. Clin, et Biol. 1969, XIV, 1054) qui fait appel à une précipitation par l'acide octanoïque (ou acide caprylique/caprylate). Celui-ci précipite la plupart des protéines du plasma et conserve dans le surnageant les immunoglobulines. La purification de ces immunoglobulines est poursuivie par adsorption (en "batch") sur un échangeur d'anions, la DEAE-cellulose, qui laisse également les immunoglobulines dans le surnageant. Celui-ci est ensuite concentré par ultrafiltration.

Cependant, ces précipitations à l'éthanol ou à l'acide caprylique peuvent provoquer une certaine dénaturation des protéines et la formation d'agrégats protéiques (notamment de polymères d'immunoglobulines), pouvant causer des réactions anaphylactiques. Il est donc nécessaire de procéder à des étapes ultérieures de traitement, par exemple à la propiolactone ou avec des agents réducteurs et alkylants, ou à pH 4, ou avec du PEG pour précipiter les agrégats. Ces étapes additionnelles tendent à diminuer le rendement en protéines plasmatiques.

Ainsi, la plupart des procédés de fabrication revendiquent un rendement en IgG compris entre 3,5 et 5,5 g d'IgG par Litre de plasma fractionné (« A modified caprylic acid method for manufacturing immunoglobulin G from human plasma with high yield and efficient virus clearance » - Vox Sang. 2006 Feb;90(2):97-104. et "A new methodology for polyvalent intravenous immunoglobulin solution production with a two-stage process of viral inactivation Brazilian Journal of Pharmaceutical Sciences" - vol. 46, n. 4, out./dez., 2010).

La mise en oeuvre d'un fractionnement éthanolique est par ailleurs relativement couteuse. En effet, le fractionnement d'un seul litre de plasma nécessite l'emploi de 2L d'éthanol. Pour le traitement de plusieurs milliers de litres de plasma, il est donc nécessaire de stocker de très grandes quantités d'éthanol directement sur le site de production, et qui plus est de réfrigérer les installations dédiées au fractionnement éthanolique. De plus les installations industrielles doivent être adaptées au stockage, au traitement, à l'élimination et éventuellement au recyclage de grands volumes de solvant ce qui représente des contraintes techniques et des coûts non négligeables.

La pureté des protéines plasmatiques est un enjeu important pour garantir la meilleure efficacité et innocuité de celles-ci lors de leur utilisation. Ainsi le fractionnement éthanolique ou caprylique doit être complété par d'autres étapes de purification pour atteindre la pureté désirée, mais ces opérations sont réalisées au détriment du rendement suite à des pertes supplémentaires de produit. Pour ces étapes additionnelles, les techniques de chromatographie sont de plus en plus utilisées car elles se caractérisent par des taux de purification importants couplés à des rendements élevés. Il est donc intéressant de positionner ces techniques de chromatographie le plus près possible du plasma de départ afin de gagner en rendement et en pureté tout en conservant l'intégrité des protéines d'intérêt. Ceci est déjà appliqué pour la capture de protéines plasmatiques peu abondantes telles que le facteur XI, le facteur IX, le facteur VIII, le facteur Von Willebrand, la protéine C l'antithrombine III etc. Mais pour les protéines abondantes telles que les immunoglobulines polyvalentes, l'albumine, le fibrinogène, les volumes de gel de chromatographie nécessaires à la capture de ces molécules seraient trop importants et aboutiraient à des outils industriels non rentables (taille des équipements et coûts de gels). Si certains décrivent l'accumulation de cycles (« Description and assessment of an industrial chromatography unit for preparing Human plasma Albumin. - Biotechnologiy of Blood Proteins » 1993, vol 227, pp. 176-181) de chromatographie pour traiter de grandes quantités de protéines, ces pratiques ne sont pas optimales et encore peu développées.

Il existe donc toujours un besoin important d'augmenter le rendement de la production de protéines plasmatiques à usage thérapeutique, et notamment des immunoglobulines, de l'albumine et/ou du fibrinogène, tout en s'assurant que le produit final présente une pureté élevée et est dépourvu de contaminants potentiellement délétères.

### Résumé de l'invention :

D'une manière générale, la présente invention a pour objet un procédé de préparation d'une fraction de protéines plasmatiques humaines purifiées avec un rendement élevé, et notamment supérieur au rendement des procédés connus de l'homme du métier. Pour cela, la demanderesse a développé un procédé de préparation d'un concentré de protéines plasmatiques humaines purifiées à partir de plasma sanguin, comprenant au moins une étape de purification par chromatographie multicolonnes, et notamment par chromatographie multicolonnes d'affinité ou échangeuse d'ions ou par interactions hydrophobes ou une combinaison de ligands chimiques (multi-modes). Plus précisément, le procédé selon l'invention propose de réaliser une étape de chromatographie en fractionnant la colonne de chromatographie habituellement utilisée en plusieurs colonnes plus petites, placées en séries ou gérées de manière indépendantes. Une telle étape mettant en oeuvre une chromatographie multicolonnes permet d'une part d'utiliser le maximum des groupements fonctionnels présents dans le gel de chromatographie et d'autre part de réduire fortement le volume de gel de chromatographie à utiliser par lot de préparation de protéines plasmatiques.

L'invention telle que définie dans les revendications a pour objet un procédé de préparation de concentrés de protéines plasmatiques purifiées à usage thérapeutique à partir de plasma sanguin comprenant des étapes de purification selon lesquelles on soumet successivement du plasma sanguin ou une fraction plasmatique à trois chromatographies multicolonnes, de manière à récupérer successivement trois concentrés de protéines plasmatiques purifiées, lesdits concentrés de protéines plasmatiques purifiées à usage thérapeutique étant un concentré d'immunoglobulines, un concentré de fibrinogène et un concentré d'albumine.

La présente invention a également pour objet un procédé de préparation d'un concentré d'immunoglobulines (Ig) humaines avec un rendement supérieur par rapport aux procédés connus de l'homme du métier. Pour cela, la demanderesse a développé un procédé de préparation d'Ig purifiées, dans lequel l'étape initiale de fractionnement à l'éthanol et/ou à l'acide caprylique, est remplacée par une étape de capture par chromatographie multicolonnes, et notamment par chromatographie multicolonnes d'affinité ou échangeuse d'ions ou par interactions hydrophobes ou une combinaison de ligands chimiques (multi-modes). Aucune étape d'élimination des contaminants protéiques par précipitation n'est mise en oeuvre avant la chromatographie. Le plasma sanguin ou cryosurnageant de plasma sanguin est directement soumis à ladite chromatographie multicolonnes. Dans le cas où la chromatographie multicolonnes est une chromatographie à échange d'anions, le procédé selon l'invention peut avantageusement comporter une étape de fractionnement à l'acide caprylique et/ou à l'éthanol à la suite de cette chromatographie.

L'invention a donc pour objet un procédé de préparation d'un concentré de protéines plasmatiques purifiées à usage thérapeutique à partir de plasma sanguin comprenant une étape de purification selon laquelle on soumet du plasma sanguin ou une fraction plasmatique à une chromatographie multicolonnes.

Notamment, l'invention a pour objet un procédé de préparation de concentrés d'immunoglobulines humaines à usage thérapeutique à partir de plasma sanguin comprenant une étape de purification des immunoglobulines selon laquelle on soumet du plasma ou du surnageant de plasma cryoprécipité à une chromatographie multicolonnes d'affinité.

Dans un autre exemple de mise en oeuvre, l'étape de purification des immunoglobulines est réalisée en soumettant du plasma ou du surnageant de plasma cryoprécipité à une chromatographie multicolonnes à échange d'anions. Il est alors possible de prévoir une étape ultérieure de purification par précipitation à l'acide caprylique à l'issue de laquelle on récupère le surnageant contenant les immunoglobulines.

L'invention a également pour objet un procédé de préparation de concentrés d'albumine et/ou de fibrinogène humains à usage thérapeutique à partir de plasma sanguin comprenant une étape de purification de l'albumine et/ou du fibrinogène selon laquelle on soumet du plasma ou du surnageant de plasma cryoprécipité à une chromatographie multicolonnes, et notamment par chromatographie multicolonnes d'affinité ou échangeuse d'ions ou par interactions hydrophobes ou une combinaison de ligands chimiques (multi-modes).

Selon un mode de réalisation particulier de l'invention, la chromatographie multicolonnes d'affinité ou échangeuse d'ions capture la protéine d'intérêt (fibrinogène et/ou albumine) qui peut ensuite être éluée. Selon un autre mode de réalisation particulier de l'invention, la chromatographie multicolonnes d'affinité ou échangeuse d'ions capture les contaminants afin de récupérer la protéine d'intérêt (albumine et/ou fibrinogène) dans la fraction non retenue. Selon l'invention, un tel procédé peut également comporter une étape additionnelle ultérieure selon laquelle on soumet la fraction contenant l'albumine et/ou le fibrinogène à une étape de purification par précipitation, à l'issue de laquelle les contaminants restent en solution.

D'une manière générale, selon l'invention, il est possible de procéder à l'étape de purification d'une protéine plasmatique à usage thérapeutique par chromatographie multicolonnes directement à partir de plasma sanguin, ou de cryosurnageant, ou d'une fraction éthanolique ou caprylique, éventuellement sécurisée viralement, ou de tout produit issu d'une étape intermédiaire de purification et notamment d'une filtration, d'une autre chromatographie, voire même d'une chromatographie multicolonne, etc.

Dans un mode de réalisation particulier de l'invention, on soumet directement du plasma ou du surnageant de plasma cryoprécipité à une première chromatographie multicolonnes pour capturer une protéine plasmatique, puis la fraction non retenue de cette chromatographie est soumise à une seconde chromatographie multicolonnes pour capturer une seconde protéine plasmatique.

Par la suite, la fraction non retenue de la deuxième chromatographie multicolonnes peut être soumise à une chromatographie, notamment chromatographie multicolonnes, ou à une étape de précipitation pour purifier une troisième protéine plasmatique.

Dans un exemple de mise en oeuvre particulier, le procédé selon l'invention consiste en un procédé de préparation d'un concentré d'albumine et/ou de fibrinogène à usage thérapeutique à partir de plasma sanguin comprenant une étape de purification de surnageant de plasma cryoprécipité par chromatographie multicolonnes d'affinité ou échangeuse d'ions, suivie éventuellement d'une étape d'élimination des contaminants présents dans la fraction comprenant l'albumine et/ou le fibrinogène par exemple par précipitation de ladite fraction à l'éthanol. Dans un autre exemple de réalisation, l'étape de purification par chromatographie multicolonnes échangeuse d'ions ou par interactions hydrophobes ou une combinaison de ligands chimiques (multi-modes) est réalisée sur une fraction plasmatique déplétée en immunoglobulines. Avantageusement, le procédé de préparation d'un concentré d'albumine et/ou de fibrinogène est mis en oeuvre à la suite d'un procédé de préparation d'un concentré d'immunoglobulines, éventuellement par chromatographie multicolonnes, en utilisant la fraction plasmatique récupérée à l'issu dudit procédé.

Ainsi, l'invention a également pour objet un procédé de fractionnement de plasma sanguin comprenant les étapes consistant à soumettre directement du plasma sanguin ou du surnageant de plasma cryoprécipité successivement à :
- une chromatographie multicolonnes par échange d'ions ou par interactions hydrophobes ou une combinaison de ligands chimiques (multi-modes) à l'issue de laquelle on récupère la fraction contenant les immunoglobulines; puis
- une chromatographie multicolonnes échangeuse d'ions ou par interactions hydrophobes ou une combinaison de ligands chimiques (multi-modes) à l'issue de laquelle on récupère la fraction contenant l'albumine ; puis
- une chromatographie multicolonnes échangeuse d'ions ou par interactions hydrophobes ou une combinaison de ligands chimiques (multi-modes) à l'issue de laquelle on récupère la fraction contenant le fibrinogène.

L'enchainement des trois étapes peut éventuellement être modifié, afin de favoriser l'extraction d'une des protéines vis-à-vis des deux autres selon le besoin.

Ainsi, l'invention a également pour objet un procédé de fractionnement de plasma sanguin comprenant les étapes consistant à soumettre directement du plasma sanguin ou du surnageant de plasma cryoprécipité successivement à :
- une chromatographie multicolonnes par échange d'ions ou par interactions hydrophobes ou une combinaison de ligands chimiques (multi-modes) à l'issue de laquelle on récupère la fraction contenant les immunoglobulines; puis
- une chromatographie multicolonnes échangeuse d'ions ou par interactions hydrophobes ou une combinaison de ligands chimiques (multi-modes) à l'issue de laquelle on récupère la fraction contenant le fibrinogène; puis
- une chromatographie multicolonnes échangeuse d'ions ou par interactions hydrophobes ou une combinaison de ligands chimiques (multi-modes) à l'issue de laquelle on récupère la fraction contenant l'albumine.

La fraction plasmatique est ainsi soumise successivement à trois chromatographies multicolonnes à l'issue desquelles elle est déplétée successivement en immunoglobulines, en albumine puis en fibrinogène, ou autre enchainement.

Dans un exemple de mise en oeuvre particulier, le procédé selon l'invention consiste en un procédé de préparation d'un concentré d'immunoglobulines à usage thérapeutique à partir de plasma sanguin comprenant successivement :
(a) une première étape d'inactivation ou élimination virale par traitement physico-chimique du plasma tel qu'un traitement au solvant/détergent ou détergent seul ou solvant seul sans limitation des combinaisons possibles des entités relatives;
(b) une étape de purification de surnageant de plasma cryoprécipité par chromatographie multicolonnes d'affinité du plasma inactivé issu de l'étape (a);
(c) éventuellement une étape d'élimination d'anticorps anti-hémagglutinines, notamment par chromatographie multicolonnes d'affinité anti-A et anti-B, du concentré d'immunoglobulines issu de l'étape (b) ;
(d) éventuellement une seconde étape d'inactivation ou élimination virale par nanofiltration du concentré d'immunoglobulines issu de l'étape (b) ou (c) ; et
(e) une étape d'addition d'un ou plusieurs stabilisants pharmaceutiquement acceptables au concentré d'immunoglobulines issu de l'étape (b), (c) ou (d).

Dans un autre exemple de mise en oeuvre particulier, le procédé selon l'invention consiste en un procédé de préparation d'un concentré d'immunoglobulines à usage thérapeutique à partir de plasma sanguin comprenant successivement :
(a') une étape de purification de surnageant de plasma cryoprécipité par chromatographie multicolonnes d'affinité ;
(b') une première étape d'inactivation ou élimination virale par solvant/détergent du concentré d'immunoglobulines obtenu à l'étape (a') ;
(c') éventuellement une étape d'élimination d'anticorps anti-A et anti-B, notamment par chromatographie multicolonnes d'affinité, du concentré d'immunoglobulines issu de l'étape (b') ;
(d') éventuellement une seconde étape d'inactivation ou élimination virale par nanofiltration du concentré d'immunoglobulines issu de l'étape (b') ou (c') ; et
(e') une étape d'addition d'un ou plusieurs stabilisants pharmaceutiquement acceptables au concentré d'immunoglobulines issu de l'étape (b'), (c') ou (d').

Dans un autre exemple de mise en oeuvre particulier, le procédé selon l'invention consiste en un procédé de préparation d'un concentré d'immunoglobulines à usage thérapeutique à partir de plasma sanguin comprenant successivement :
(A) une étape de fractionnement éthanolique et/ou caprylique de surnageant de plasma cryoprécipité;
(B) éventuellement une première étape d'inactivation ou élimination virale par solvant/détergent de la solution obtenue à l'étape (A) ;
(C) une étape de purification par chromatographie multicolonnes à échange d'anions de la solution obtenue à l'étape (A) ou (B);
(D) éventuellement une étape d'élimination d'anticorps anti-A et anti-B, notamment par chromatographie multicolonnes d'affinité, du concentré d'immunoglobulines obtenu à l'issu de l'étape (C) ;
(E) éventuellement une seconde étape d'inactivation ou élimination virale par nanofiltration du concentré d'immunoglobulines obtenu à l'issu de l'étape (C) ou (D) ; et
(F) une étape d'addition d'un ou plusieurs stabilisants pharmaceutiquement acceptables au concentré d'immunoglobulines issu de l'étape (C), (D) ou (E).

L'invention a également pour objet un procédé de fractionnement de plasma sanguin comprenant les étapes consistant à soumettre directement du plasma sanguin ou du surnageant de plasma cryoprécipité à :
- une chromatographie multicolonnes d'affinité dans laquelle au moins un ligand d'affinité est un ligand liant spécifiquement les immunoglobulines, à l'issue de laquelle on récupère la fraction contenant les immunoglobulines; et/ou
- une chromatographie multicolonnes échangeuse d'ions, par interactions hydrophobes ou une combinaison de ligands chimiques (multi-modes) dite mode mixte/multimodale (« mixed-mode chromatography »), optionnellement hautement tolérante au sel (« salt tolerant »), à l'issue de laquelle on récupère la fraction contenant le fibrinogène et/ou l'albumine.

L'invention a aussi pour objet un procédé de fractionnement de plasma sanguin comprenant les étapes consistant à soumettre directement du plasma sanguin ou du surnageant de plasma cryoprécipité à :
- une chromatographie multicolonnes d'affinité dans laquelle au moins un ligand d'affinité est un ligand liant spécifiquement les immunoglobulines, à l'issue de laquelle on récupère la fraction contenant les immunoglobulines; et/ou
- une chromatographie multicolonnes d'affinité dans laquelle au moins un ligand d'affinité est un ligand liant spécifiquement le fibrinogène, à l'issue de laquelle on récupère la fraction contenant le fibrinogène ; et/ou
- une chromatographie multicolonnes échangeuse d'ions, par interactions hydrophobes ou une combinaison de ligands chimiques (multi-modes) dite mode mixte/multimodale (« mixed-mode chromatography »), optionnellement hautement tolérante au sel (« salt tolerant »), à l'issue de laquelle on récupère la fraction contenant l'albumine.

Avantageusement, la chromatographie multicolonnes d'affinité ciblant les immunoglobulines est réalisée en premier, la ou les chromatographies multicolonnes d'affinité et d'échange d'ions ciblant le fibrinogène et/ou l'albumine étant réalisées à partir de la fraction issue de cette première étape de fractionnement et appauvrie en immunoglobulines de type G. La chromatographie multicolonnes de capture de l'albumine peut être réalisée de façon avantageuse en troisième position.

### Brève description des figures

La Figure 1 (Figures 1A-1D) représente de manière schématique le principe de la chromatographie multicolonnes tel que mis en oeuvre dans le procédé selon l'invention ;
La Figure 2 représente de manière schématique l'enchainement des étapes pour la purification en continu de protéines abondantes du plasma au moyen d'une succession de chromatographies multicolonnes, selon un exemple de mise en oeuvre du procédé selonl'invention ;
La Figure 3 est une image d'un gel SDS-PAGE en conditions non réductrice et coloration bleu de Coomassie des fractions obtenues à l'issue des chromatographies multicolonnes réalisées dans les exemples 2, 3 et 4.

### Description détaillée

### Définition

Par « protéine plasmatique », on entend selon l'invention toute protéine, et plus particulièrement toute protéine d'intérêt industriel ou thérapeutique, contenue dans le plasma sanguin. Les protéines du plasma sanguin englobent l'albumine, alpha/macroglobuline, antichymotrypsine, antithrombine, antitrypsine, Apo A, Apo B, Apo C, Apo D, Apo E, Apo F, Apo G, beta XIIa, C 1-inhibiteur, protéine C-réactive, C7, C1r, C1s, C2 C3, C4, C4bP, C5, C6, Clq, C8, C9, carboxypeptidase N, céruloplasmine, facteur B, facteur D, facteur H, facteur I, facteur IX, facteur V, facteur VII, facteur VIIa, facteur VIII, facteur X, facteur XI, facteur XII, facteur XIII, fibrinogène, fibronectine, haptoglobine, hemopexine, héparine cofacteur II, histidine rich GP, IgA, IgD, IgE, IgG, ITI, IgM, kininase II, kininogène HPM, lysozyme, PAI 2, PAI I, PCI, plasmine, inhibiteur de la plasmine, plasminogène, préalbumine, prékallicréine, properdine, protéase nexine INH, protéine C, protéine S, protéine Z, prothrombine, sérum amyloïde protéine (SAP), TFPI, thiol-protéinase, thrombomoduline, facteur tissulaire (TF), TPA, transcolabamine II, transcortine, transferrine, vitronectine, et le facteur de Willebrand.

Notamment, les protéines plasmatiques englobent les protéines de la coagulation, c'est-à-dire les protéines plasmatiques impliquées dans la chaîne de réactions en cascade aboutissant à la formation d'un caillot sanguin. Les protéines de la coagulation englobent le facteur I (fibrinogène), le facteur II (prothrombine), le facteur V (proaccélérine), le facteur VII (proconvertine), le facteur VIII (facteur anti-hémophilique A), le facteur IX (facteur anti-hémophilique B), le facteur X (facteur Stuart), le facteur XI (facteur Rosenthal ou PTA), le facteur XII (facteur Hageman), le facteur XIII (facteur stabilisant la fibrine ou FSF), la PK (Prékallicréine) le KHPM (kininogène de haut poids moléculaire), le facteur III (thromboplastine ou facteur tissulaire), le cofacteur II de l'héparine (HCII), la protéine C (PC), la thrombomoduline (TM), la protéine S (PS), le facteur de Willebrand (Wf) et l'inhibiteur de la voie du facteur tissulaire (TFPI), ou encore les facteurs tissulaires.

Dans certains modes de réalisation, la protéine plasmatique consiste en une protéine de la coagulation à activité enzymatique. Les protéines de la coagulation à activité enzymatique englobent les formes activées du facteur II (prothrombine), facteur VII (proconvertine), facteur IX (facteur anti-hémophilique B), facteur X (facteur Stuart), facteur XI (Facteur Rosenthal ou PTA), facteur XII (facteur Hageman), facteur XIII (facteur stabilisant la fibrine ou FSF) et de la PK (Prékallicréine).

Dans le contexte de l'invention, on entend par « Immunoglobulines humaines » ou « Ig humaines » des immunoglobulines polyvalentes qui peuvent être des immunoglobulines A (IgA), des immunoglobulines E (IgE), des immunoglobulines M (IgM) ou des immunoglobulines G (IgG). Les immunoglobulines humaines selon l'invention sont avantageusement des IgG, quelle que soit leur sous-classe (IgG1, IgG2, IgG3 et IgG4). Il peut s'agir d'immunoglobulines entières, ou de toute fraction intermédiaire obtenue au cours du procédé de fabrication des immunoglobulines polyvalentes.

Par « fraction plasmatique », on entend toute partie ou sous-partie du plasma, ayant fait l'objet d'une ou plusieurs étapes de purification. Les fractions plasmatiques incluent ainsi le surnageant de plasma cryoprécipité, le cryoprécipité de plasma (remis en suspension), les fractions I à V obtenues par fractionnement éthanolique (selon la méthode de Cohn ou de Kistler & Nitschmann), le surnageant et le précipité obtenus après précipitation à l'acide caprylique et/ou caprylate, les éluats de chromatographies et les fractions non adsorbées des colonnes de chromatographie, y compris des chromatographies multicolonnes, et les filtrats.

Selon l'invention, le « surnageant de plasma cryoprécipité », ou « cryosurnageant », correspond à la phase liquide obtenue après décongélation de plasma congelé (cryoprécipitation). Notamment, le cryosurnageant peut être obtenu par congélation de plasma sanguin à une température comprise entre - 10°C et - 40°C, puis décongélation douce à une température comprise entre 0°C et +6°C, préférentiellement entre 0°C et +1°C, suivie d'une centrifugation du plasma décongelé pour séparer le cryoprécipité et le cryosurnageant. Le cryoprécipité est concentré en fibrinogène, fibronectine, facteur von Willebrand et facteur VIII, tandis que le cryosurnageant contient les Facteurs du complément, les facteurs vitamine K dépendants tels que la protéine C, la protéine S, la protéine Z, le facteur II, le facteur VII, le facteur IX et le facteur X, du Fibrinogène et les immunoglobulines et l'albumine.

Par « étape de purification », on entend toute étape d'un procédé permettant l'enrichissement d'un produit d'intérêt, et notamment une protéine plasmatique, dans une fraction donnée.

Le procédé de préparation selon l'invention repose principalement sur l'emploi d'une étape de chromatographie multicolonnes pour la purification d'une protéine plasmatique à usage thérapeutique à partir de plasma sanguin ou de toute fraction plasmatique. La mise en oeuvre d'une telle étape de chromatographie multicolonnes est particulièrement adaptée pour la préparation d'un concentré d'immunoglobulines. En effet, il est possible, selon l'invention, de soumettre directement du plasma sanguin humain ou du cryosurnageant de plasma sanguin humain à une chromatographie multicolonnes. Bien entendu, il est également possible de prévoir une étape de filtration et/ou fractionnement éthanolique/caprylique en amont de la purification par chromatographie multicolonnes.

L'emploi d'une telle chromatographie multicolonnes permet d'une manière générale une diminution à l'échelle industrielle du prix de revient des protéines plasmatiques à usage thérapeutique. Notamment, la chromatographie multicolonnes permet d'utiliser le support chromatographique à saturation totale de leur capacité ce qui requière une quantité de gel moindre qu'une chromatographie classique dont l'usage habituel est de limiter la capacité dès la détection de 10% de fuite en molécule d'intérêt. De même, les phases d'élution, lavage et sanitisation étant réalisées sur des colonnes plus petites, les besoins en solutions et tampons sont largement réduits par rapport à une chromatographie classique. Si, le principe de fractionnement des molécules plasmatique par des chromatographies en cascade a déjà été décrit (John Curling et al. «A comparative study of Cohn and chromatographic fractionation using a novel affinity "Cascade Process " » PPB congres May 2005), il n'a qu'un intérêt industriel limité du fait des faibles capacités et productivités associées. De manière particulièrement surprenante et avantageuse, l'utilisation de la chromatographie multicolonnes selon l'invention rend le principe de la chromatographie en cascade beaucoup plus attractif, en permettant une meilleure capacité de capture et/ou une meilleure productivité exprimée en unité de masse de produit extrait par unité de temps et volume de gel.

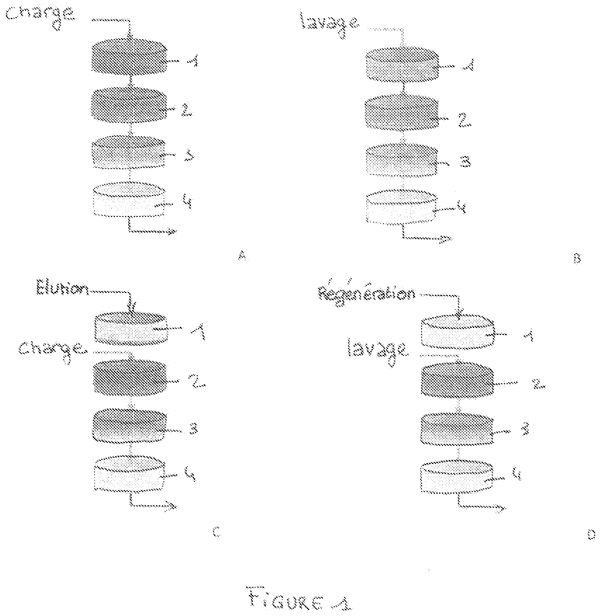

Le principe de la chromatographie multicolonnes est représenté de manière schématique à la figure 1. Une telle chromatographie multicolonnes permet le fractionnement d'une colonne (un support de chromatographie) classiquement utilisée en chromatographie, en plusieurs colonnes (du même support de chromatographie) de taille plus petite et reliées entre elles de manière à ce que la sortie de l'une soit connectée à l'entrée de l'autre.

De manière générale, la chromatographie multicolonnes exclut la juxtaposition (en série ou en parallèle) de plusieurs colonnes de chromatographies de natures différentes.

En résumé, la fraction à purifier est injectée sur une colonne 1 (figure 1A), dont la sortie est reliée en série à une colonne 2. Ainsi, les fuites de la protéine plasmatique d'intérêt sortant de la colonne 1, au lieu de partir dans une fraction collectée dite non adsorbée, sont récupérées sur la colonne 2 (colonne de garde) sur laquelle la protéine plasmatique d'intérêt résiduelle va pouvoir s'adsorber. Le nombre de colonnes de garde 2, 3, 4, etc... placées en série à la suite de la colonne 1 dépend de la proportion de fuite de protéine d'intérêt à récupérer (dans l'exemple décrit ici 3 colonnes de garde 2-4 sont représentées). Une fois la charge puis le lavage de la colonne 1 terminés (figures 1A et 1B), elle peut être séparée des autres colonnes 2-4, afin de subir individuellement des étapes d'élution (figure 1C), régénération (figures 1D) et éventuellement équilibrage (non représenté). Une fois la colonne 1 rééquilibrée, elle peut être replacée en série, en tant que dernière colonne de garde, etc.

En parallèle, la colonne 2, partiellement chargée, passe alors en première position et subit à son tour une charge en fraction plasmatique dont les fuites sont de nouveau récupérées sur les colonnes de garde 3 et 4 suivantes, et ainsi de suite.

La chromatographie multicolonnes permet ainsi d'optimiser la saturation du gel sans perdre de produit par perçage, car les fuites sont récupérées sur les colonnes de garde.

Selon l'invention, plusieurs techniques de chromatographie multicolonnes peuvent être utilisées. On connait notamment la technologie « Lit Mobile Simulé » ou « SMB » (en anglais : « Simulated Moving Bed ») dont dérive la technologie SMCC (en anglais : « Sequential Multicolumn Chromatography ») qui sont particulièrement adaptées à la mise en oeuvre du procédé selon l'invention. Des exemples de mise en oeuvre de chromatographies multicolonnes sont décrits dans les demandes de brevet WO2007/144476 et WO2009/122281.

Selon l'invention, la chromatographie multicolonnes peut être une chromatographie d'affinité, échangeuse d'ions (anions ou cations), hydrophobe, en mode mixte ou à exclusion de taille. Préférentiellement, la chromatographie multicolonnes est une chromatographie multicolonnes d'affinité, mode mixte ou échangeuse d'anions.

Dans un exemple de mise en oeuvre particulier de l'invention, la chromatographie multicolonnes est une chromatographie radiale, c'est-à-dire utilisant des colonnes radiales. Selon l'invention, il est possible d'utiliser des colonnes radiales présentant un ratio d'au moins deux entre la surface du diamètre extérieur (côté entrée) plus grande et la surface du diamètre intérieur (côté sortie) plus petite. Ces colonnes permettent d'augmenter les débits de chargements, rendant le procédé particulièrement avantageux lorsqu'il y a des volumes de plasma importants à traiter et/ou que la protéine plasmatique d'intérêt, telle que les immunoglobulines, doit être purifiée rapidement afin de conserver son intégrité moléculaire. De même, cela permet de réduire la durée des étapes de purification et ainsi de multiplier les lots par unité de temps.

Selon les besoins, et plus particulièrement les volumes de plasma à traiter, on peut utiliser des colonnes de chromatographie de quelques millilitres (pour une mise en oeuvre à l'échelle du laboratoire par exemple) par exemple de 5 à 20 mL, à plusieurs centaines ou milliers de litres (à l'échelle industrielle), par exemple de 200 à 2000 L. Pour chaque gamme de colonnes, il est également possible d'adapter la hauteur de lit de gel formant la phase stationnaire, par exemple 6 cm, 12 cm ou 18 cm. L'homme du métier sait, en fonction des volumes à traiter et/ou du débit souhaité, adapter le nombre de colonnes, leurs dimensions et les hauteurs de gel.

Dans un exemple particulier, et notamment pour la préparation d'un concentré d'immunoglobulines directement à partir de cryosurnageant, on utilise avantageusement une chromatographie multicolonnes d'affinité.

Il est connu que la plupart des gels d'affinité (formant la phase stationnaire de la chromatographie d'affinité), notamment ceux utilisés industriellement, possèdent une capacité de charge maximale en immunoglobulines (Ig) quatre à cinq fois plus faible qu'un gel à échange d'ions (20-30 g.L-1 contre 80-100 g.L-1). Il est donc généralement admis que pour traiter des volumes de plasma importants la quantité de gel d'affinité à utiliser est telle que son utilisation est économiquement inadaptée.

Cependant, la Demanderesse a découvert que ce désavantage peut être contré en fractionnant la colonne de chromatographie en plusieurs colonnes plus petites et notamment en 3 à 8 colonnes, préférentiellement 3 à 5 colonnes. L'utilisation d'une pluralité de colonnes de chromatographie permet de placer en série d'une (de) colonne(s) principale(s) des colonnes de garde qui vont capturer les protéines plasmatiques d'intérêt qui ont fui de la (es) colonne(s) principale(s) lors du chargement de la protéine d'intérêt. Les ligands d'affinités présents dans cette (es) colonne(s) principale(s) sont alors saturés de manière à atteindre la capacité maximale du gel de chromatographie. Cette (es) colonne(s) est (sont) ensuite sortie(s) du circuit pour être éluée(s) séparément et permettre la récupération des protéines d'intérêt. Les colonnes de garde devenant à leur tour la (les) colonne(s) principale(s). Des cycles successifs au nombre de 3 à 50, préférablement de 5 à 30 et de façon préférée de 7 à 15 permettent de maximiser l'utilisation des ligands d'affinité et ainsi de réduire le volume total de gel nécessaire pour la capture et l'élution de la molécule d'intérêt.

Avantageusement, le ligand d'affinité est choisi parmi les anticorps, les fragments d'anticorps, les dérivés d'anticorps ou des ligands chimiques tels que des peptides, des peptides mimétiques, des peptoïdes, des nanofitines ou encore des ligands oligonucléotidiques tels que les aptamères.

Par exemple, on peut utiliser un gel comprenant une matrice d'agarose réticulée, permettant de travailler à débits élevés, en combinaison avec un ligand d'affinité apte à fixer la protéine d'intérêt. Dans un mode de réalisation particulier, le ligand d'affinité permet de lier le fragment Fc des Ig humaines ou toute séquence conservée au sein des immunoglobulines.

Dans un autre mode de réalisation particulier, le ligand d'affinité utilisé est une protéine recombinante reconnaissant le fragment Fc des Ig et couplée à une matrice, par exemple le gel IgSelect de GE Healthcare.

Avantageusement, le ligand peut être choisi de manière à reconnaitre spécifiquement une classe d'immunoglobulines (par exemple les immunoglobulines G) et/ou à reconnaitre spécifiquement une ou plusieurs sous-classe d'immunoglobulines (par exemple IgG1 et/ou IgG2 et/ou IgG3 et/ou IgG4). Dans un autre mode de réalisation particulier, le ligand d'affinité utilisé est la protéine G, qui possède une affinité pour les IgG et ne présente avantageusement pas d'affinité pour les IgA. Dans encore un autre mode de réalisation particulier, le ligand d'affinité utilisé est la protéine A qui lie spécifiquement les IgG1, IgG2 et IgG4 mais ne permet pas de capturer les IgG3.

Dans un exemple de réalisation particulier, le ligand d'affinité de la chromatographie multicolonnes d'affinité présente une affinité pour les immunoglobulines G, et est avantageusement choisi parmi les ligands présentant une affinité pour les IgG1, IgG2, IgG3 et IgG4. Plus particulièrement, le concentré d'IgG obtenu selon le procédé de l'invention présente avantageusement un profil de répartition des sous-classes d'IgG similaire à celui du plasma. Notamment, le concentré d'IgG selon l'invention comporte avantageusement entre 50 et 70% d'IgG1, 25 à 35% d'IgG2, 2 à 8% d'IgG3 et 1 à 8% d'IgG4 ou de façon encore plus préférée entre 60 et 70% d'IgG1, 30 à 35% d'IgG2, 3 à 6% d'IgG3 et 2 à 5% d'IgG4. Dans un mode de réalisation particulier, le concentré obtenu selon le procédé de l'invention peut présenter une faible diminution des sous-classes IgG3 et/ou IgG4 par rapport au plasma, le produit restant néanmoins comparable en efficacité thérapeutique à un produit ayant un répertoire de sous-classes d'IgG similaire à celui du plasma.

Dans un autre exemple de réalisation particulier, le ligand d'affinité de la chromatographie multicolonnes d'affinité présente une affinité pour le fibrinogène ou l'albumine. Plus particulièrement, l'invention a également pour objet un procédé de préparation de concentrés d'albumine et/ou de fibrinogène humain directement à partir de plasma sanguin, selon lequel on soumet ledit plasma ou du surnageant de plasma cryoprécipité à une chromatographie multicolonne d'affinité dans laquelle le(s) ligand(s) d'affinité présente(nt) une affinité pour le fibrinogène et/ou l'albumine. Le ligand utilisé peut être notamment tout ligand commercialement disponible, par exemple le ligand d'affinité CaptureSelect HSA (Life Technologies) ou le ligand de la matrice d'affinité CaptureSelect Fibrinogen (Life Technologies).

De manière avantageuse, le ligand d'affinité tel que choisi pour le procédé de l'invention est résistant aux conditions de sanitisation et/ou de réutilisation intensive compatibles avec une utilisation industrielle. Dans un mode de réalisation particulier, le ligand d'affinité est ainsi avantageusement choisi parmi les peptides et/ou peptoïdes (issus de biologie combinatoire, telle que le phage display), les nanofitines (extraites de bactéries extrêmophiles), et/ou les aptamères.

Dans un exemple particulier, le ligand d'affinité est un aptamère, et notamment un aptamère nucléique. Le terme « aptamère » tel qu'utilisé ici désigne une molécule d'acide nucléique monobrin, ADN ou ARN, et notamment une molécule d'acide nucléique monobrin capable de se lier de manière spécifique à la protéine d'intérêt, par exemple à une immunoglobuline en se liant au fragment Fc des Ig humaines ou aux séquences conservées de l'immunoglobuline. Avantageusement, l'aptamère peut être choisi de manière à reconnaitre spécifiquement une classe d'immunoglobuline (par exemple les immunoglobulines G) et/ou à reconnaitre spécifiquement une ou plusieurs sous-classe d'immunoglobuline (par exemple IgG1 et/ou IgG2 et/ou IgG3 et/ou IgG4). Les aptamères comprennent généralement entre 5 et 120 nucléotides et peuvent être sélectionnés in vitro selon un procédé connu sous le nom de SELEX (Systematic Evolution of Ligands by Exponential Enrichment). Par « aptamère nucléique », on entend selon l'invention un acide nucléique simple brin, et notamment acide nucléique simple brin se liant spécifiquement au fragment Fc des IgG humaines.

Les aptamères présentent de nombreux avantages. De par leur nature oligonucléotidique, les aptamères possèdent une faible immunogénicité et une résistance importante à des conditions physico-chimiques stringentes (présence de DMSO, d'un pH très acide ou très basique, utilisation de solvants organiques ou de température élevée) permettant des stratégies de sanitisation variées dans le cadre d'un usage en tant que ligand d'affinité. Il est ainsi possible d'augmenter la durée de vie des gels d'affinité en procédant à des étapes de sanitisation pour nettoyer les colonnes de chromatographie et limiter leur encrassement et réduire les risques de contamination virale ou prion, et ce malgré les multiples cycles et les charges augmentées par rapport à une chromatographie classique. Les aptamères, grâce à leur structure de type acide nucléique, sont particulièrement adaptées aux sanitisations à pH basique, permettant des réutilisations de 100 à 200 cycles.

La demande de brevet FR 2 970 003, au nom de la Demanderesse, décrit des procédés de fabrication de supports d'affinité avec des aptamères nucléiques immobilisés. Notamment, cette demande décrit un procédé d'immobilisation d'acides nucléiques comprenant au moins une fonction amine réactive, par greffage sur un support solide présentant à sa surface des groupes acides carboxyliques activés.

Dans un mode de réalisation particulier, la chromatographie multicolonnes peut être avantageusement appliquée à une ou plusieurs étapes de purification successives, utilisant des colonnes de chromatographie successives. Ainsi, la fraction non retenue de la première chromatographie multicolonnes peut être utilisée pour purifier les autres protéines plasmatiques d'intérêt dans l'ordre le plus approprié. Dans un exemple de mise en oeuvre particulier, une première chromatographie multicolonnes est réalisée à partir de plasma sanguin ou de cryoprécipité pour purifier les immunoglobulines. La fraction de plasma issue de cette première purification est soumise à une seconde chromatographie multicolonnes pour purifier l'albumine. La fraction de plasma issue de cette seconde chromatographie multicolonnes, déplétée en immunoglobulines et en albumine, peut ensuite être soumise à une troisième chromatographie multicolonnes pour purifier le fibrinogène, etc. Il est notamment possible de traiter le plasma en flux continu, pour purifier en continu l'immunoglobuline, l'albumine et/ou le fibrinogène notamment, sans activer la cascade de coagulation.

Dans un autre exemple de mise en oeuvre particulier, une première chromatographie multicolonnes est réalisée à partir de plasma sanguin ou de cryoprécipité pour purifier les immunoglobulines. La fraction de plasma issue de cette première purification est soumise à une seconde chromatographie multicolonnes pour purifier le fibrinogène. La fraction de plasma issue de cette seconde chromatographie multicolonnes, déplétée en immunoglobulines et en fibrinogène, peut ensuite être soumise à une troisième chromatographie multicolonnes pour purifier l'albumine, etc. Il est notamment possible de traiter le plasma en flux continu, pour purifier en continu l'immunoglobuline, le fibrinogène et/ou l'albumine notamment, sans activer la cascade de coagulation.

Selon l'invention, le procédé de préparation de concentrés de protéines plasmatiques humaines à usage thérapeutique peut également comporter au moins une des étapes suivantes :
(i) une étape d'inactivation virale ou d'élimination virale ;
(ii) une étape de chromatographie à échange d'ions ;
(iii) une étape d'élimination d'anticorps anti-A et anti-B, notamment par chromatographie d'affinité ;
(iv) une étape de précipitation à l'acide caprylique et/ou caprylate ;
(v) une étape de concentration par ultrafiltration ;
(vi) une étape de formulation.

Avantageusement, le procédé de préparation selon l'invention permet d'obtenir un concentré de protéines humaines à usage thérapeutique et peut comporter au moins une étape ultérieure parmi les étapes (i) à (vi).

Dans un exemple de réalisation particulier, le procédé de préparation permet d'obtenir un concentré d'immunoglobulines humaines à usage thérapeutique, comportant éventuellement une ou plusieurs étapes ultérieures parmi les étapes (i) à (vi).

Dans un autre exemple de réalisation particulier, le procédé de préparation permet d'obtenir un concentré d'albumine à usage thérapeutique, comportant éventuellement une ou plusieurs étapes ultérieures parmi les étapes (i) à (vi).

Dans un autre exemple de réalisation particulier, le procédé de préparation permet d'obtenir un concentré de fibrinogène à usage thérapeutique, comportant éventuellement une ou plusieurs étapes ultérieures parmi les étapes (i) à (vi).

Par exemple, le concentré de protéines plasmatiques humaines et/ou le concentré d'immunoglobulines obtenu en soumettant directement du plasma sanguin ou du cryosurnageant à une chromatographie multicolonnes, d'affinité ou échangeuse d'anions notamment, peut avantageusement subir au moins une étape d'élimination ou d'inactivation d'au moins un agent infectieux.

Parmi les agents infectieux visés à l'étape (i), on peut citer les virus et les ATNC (agents transmissibles non conventionnels) comme le prion.

Une inactivation virale comprend souvent un traitement avec des produits chimiques, par exemple par solvant, et/ou détergent et/ou par la chaleur (pasteurisation et/ou chauffage à sec) et/ou par irradiation (Gamma et/ou par les UVC) et/ou par traitement pH (traitement à pH acide).

De préférence, l'étape (i) selon l'invention comprend au moins un traitement par solvant et détergent. Le traitement par solvant et détergent (appelé généralement traitement Solvant/Détergent ou S/D) comprend notamment le traitement par le tri-n-butylphosphate (TnBP) et/ou un détergent qui est choisi parmi le Triton X-100, le Tween (de préférence Tween 80), le cholate de sodium et le 2-[4-(2,4,4-triméthylpentane-2- yl)phénoxy]éthanol (Octoxinol).

Une nanofiltration peut également être utilisée pour éliminer les agents infectieux, notamment les virus et les ATNC. Dans le cas des protéines plasmatiques, la nanofiltration se réfère généralement à la filtration du concentré de protéines d'intérêt à travers un filtre avec une taille de pores inférieure à 80 nm. Les filtres disponibles sont par exemple les filtres BioEx, Planova® 75 nm, Planova® 35 nm, Planova® 20 nm ou Planova® 15 nm (Asahi corporation), Ultipor DV 50 or DV 20 (Pall corporation), Virosart CPV (Sartorius), Viresolve NFR ou NFP (Millipore). La nanofiltration peut être avantageusement effectuée sur un filtre unique ou sur plusieurs filtres en série de porosité identique ou décroissante.

L'élimination des agents infectieux peut également être réalisée au moyen d'une filtration en profondeur. Les filtres disponibles sont par exemple des filtres composés de cellulose régénérée, dans lesquels des adjuvants de filtration peuvent avoir été additionnés (tels que la cellite, la perlite ou des terres de Kieselguhr) commercialisés par Cuno (filtres Zeta+ VR series), Pall-Seitz (P-series Depth Filter) ou Sartorius (Virosart CPV, Sartoclear P depth filters).

Dans un mode de réalisation particulier, une étape d'inactivation virale peut être avantageusement réalisée directement sur le plasma ou le surnageant de plasma cryoprécipité ou le cryoprécipité de plasma remis en suspension, afin que l'ensemble des protéines à purifier bénéficient du traitement en amont de la chromatographie multicolonnes.

Dans un autre mode de réalisation particulier, le plasma brut et/ou le surnageant de plasma cryoprécipité et/ou le cryoprécipité de plasma remis en suspension, peuvent être soumis à une filtration en profondeur ou à une séquence de filtration, par exemple sur filtre polypropylène ou média équivalent de 6µm, 1 µm, puis 0,45 - 0,2 µm, en amont de la chromatographie multicolonnes. Une telle étape préliminaire permet avantageusement de prévenir et/ou réduire un encrassement prématuré des colonnes utilisées pour la chromatographie multicolonnes. Dans un autre mode de réalisation particulier, le ou les filtres profondeur utilisés sont des filtres profondeur délipidants (par exemple Cuno ou Pall-Seitz) permettant une réduction des lipides dans la fraction plasmatique traitée.

Avantageusement, l'étape (i) d'inactivation ou élimination virale est suivie d'une étape (ii) de chromatographie à échange d'ions.

Dans un exemple particulier, l'étape (ii) de chromatographie à échange d'ions est une chromatographie à échange d'anions ou de cations. De telles étapes de chromatographie à échange d'anions ou de cations sont décrites dans les demandes de brevets EP 0 703 922 et WO 99/64462 au nom de la Demanderesse.

Dans un exemple particulier, l'étape (ii) de chromatographie à échange d'anions peut être mise en oeuvre sur un gel de polysaccharide réticulé ou de polymère vinylique ou acrylique, greffé de groupements DEAE ou TMAE ou QAE, comme décrit dans les demandes de brevet WO2002/092632 et WO2013/007740 au nom de la Demanderesse.

Dans un autre exemple particulier, la chromatographie multicolonnes à échange d'anions et/ou en mode mixte est réalisée sur une matrice à haute tolérance au sel permettant de capturer la protéine plasmatique dans un milieu ayant une salinité élevée tel que le plasma ou la fraction non retenue d'une chromatographie. Avantageusement, la chromatographie multicolonnes à échange d'anions et/ou mode mixte à haute tolérance au sel est réalisée sur le gel STAr AX de PALL BIOSEPRA, Capto MMC de GE Healthcare ou le gel ESHMUNO HCX de Merck ou tout gel équivalent connu de l'homme du métier.

Notamment, pour la préparation d'un concentré d'immunoglobulines à usage thérapeutique, l'étape (ii) de chromatographie à échange d'anions peut comprendre :
- l'ajustement à un pH de 8 à 10 de la solution ayant subi le traitement au solvant-détergent (étape (i)) ;
- sa charge sur la colonne de chromatographie préalablement équilibrée en tampon à pH 8 à 10 ce qui permet l'adsorption des immunoglobulines et le passage des protéines non adsorbées dans l'effluent ;
- un lavage par le même tampon jusqu'à élimination de toutes les protéines non adsorbées et du mélange solvant détergent ;
- et l'élution des immunoglobulines par un tampon approprié.

Cette élution peut être réalisée par du tampon phosphate à pH compris entre 4 et 7, et de préférence à pH 6,2 pour éluer les immunoglobulines.

Le procédé selon l'invention peut également comporter une étape (iii) d'élimination d'anticorps anti-A et/ou anti-B. Dans un exemple particulier, l'étape (iii) d'élimination d'anticorps anti-A et/ou anti-B est réalisée sur la solution obtenue à l'issue de l'étape (ii). Cette étape peut par exemple être effectuée selon la méthode décrite dans la demande de brevet WO 2007/077365 au nom de la Demanderesse. Ainsi, dans le cas de la préparation d'un concentré d'immunoglobulines à usage thérapeutique, la solution obtenue à l'étape (ii) peut être soumise à une étape d'élimination des anticorps anti-A et anti-B par chromatographie d'immunoaffinité par percolation dudit concentré d'immunoglobulines polyvalentes sur un support dont la matrice est greffée de groupes oligosaccharides antigéniquement similaires aux groupes sanguins A et/ou B, ou sur un mélange de supports dont les matrices sont greffées de groupes oligosaccharides antigéniquement similaires aux groupes sanguins A et/ou B. Dans un mode de réalisation particulier, l'étape (iii) d'élimination d'anticorps anti-A et/ou anti-B est réalisée sur une chromatographie multicolonnes d'affinité.

Dans certain cas, le procédé selon l'invention peut comporter une étape (iv) de précipitation à l'acide caprylique et/ou caprylate. D'une manière générale, l'addition d'acide caprylique et/ou caprylate, en milieu légèrement acide permet de précipiter les protéines plasmatiques, à l'exception des Ig qui restent dans le surnageant. Selon l'invention, il est possible de procéder à cette étape (iv) de précipitation caprylique avant ou après l'étape de purification par chromatographie multicolonnes.

Dans un exemple de mise en oeuvre particulier, l'étape de purification de la protéine plasmatique d'intérêt par chromatographie multicolonnes échangeuse d'anions, est suivie d'une étape ultérieure de précipitation à l'acide caprylique et/ou caprylate. Une telle combinaison chromatographie multicolonnes échangeuse d'ions/précipitation à l'acide caprylique peut être particulièrement avantageuse pour la préparation d'un concentré d'Ig (et tout particulièrement d'IgG) et/ou un concentré d'albumine et/ou fibrinogène.

Notamment, pour la préparation d'un concentré d'immunoglobulines à usage thérapeutique, l'étape (iv) de précipitation à l'acide caprylique peut comprendre les étapes consistant à
- Eventuellement ajuster le pH de la fraction plasmatique à une valeur comprise entre 3.0 et 6.0, préférentiellement à pH 4 ;
- Ajouter l'acide caprylique à la fraction plasmatique ;
- Centrifuger ou filtrer pour récupérer le surnageant enrichi en IgG.

Le procédé selon l'invention peut avantageusement comporter une ou plusieurs étapes (v) de concentration par ultrafiltration. Par exemple, lors de la préparation d'un concentré d'Ig, il est possible de soumettre la fraction enrichie en Ig, issue de l'étape de chromatographie multicolonnes, éventuellement préalablement soumise à l'une et/ou l'autre des étapes (i) à (iv), à une ultrafiltration sur membrane.

Il est également possible de prévoir une étape (vi) d'addition d'un ou plusieurs stabilisants pharmaceutiquement acceptables au concentré de protéines plasmatiques humaines. Selon l'invention, il est possible de prévoir une étape (i) de sécurisation virale additionnelle, par nanofiltration, avant l'étape (vi) de formulation.

Par stabilisants pharmaceutiquement acceptables, on entend les formulations adaptées aux concentrés de protéines plasmatiques notamment les excipients tels que décrits dans les demandes FR 03 08403, et préférentiellement les formulations adaptées aux concentrés d'immunoglobulines, et notamment les excipients tels que décrits dans les demandes FR 03 04388, FR 08 59117, FR 10 54721, FR 10 55825 au nom de la Demanderesse.

L'étape (vi) de formulation peut éventuellement être suivie d'une étape (vii) de congélation ou de lyophilisation de ladite préparation pharmaceutique obtenue à l'étape (vi).

Avantageusement, le procédé selon l'invention permet la préparation de concentrés d'immunoglobulines, et/ou de fibrinogène et/ou d'albumine, notamment en évitant des étapes de stockage à froid.

Avantageusement, le procédé selon l'invention permet l'obtention d'un concentré d'immunoglobulines avec un rendement supérieur à 5g.L-1 de plasma, de manière préférée avec un rendement supérieur à 6g.L-1 de plasma. De manière très avantageuse, le procédé selon l'invention est optimisé afin d'obtenir un rendement en immunoglobulines proche de 7-8 g.L-1 de plasma initial.

De manière avantageuse, le procédé selon l'invention permet l'obtention d'un concentré d'albumine avec un rendement supérieur à 30 g.L-1, de manière très avantageusement supérieure à 35g.L-1. En ce qui concerne le fibrinogène le taux plasmatique étant de l'ordre de 3 g/L une étape de capture par affinité permet avantageusement d'en capturer 80% en une étape. De manière préférée la chromatographie continue permet d'atteindre des rendements supérieurs à 90%.

De manière avantageuse, le concentré d'immunoglobulines obtenu par le procédé selon l'invention possède un répertoire antigénique similaire ou identique au répertoire antigénique du plasma. Dans un mode de réalisation particulier, le concentré d'immunoglobulines obtenu par le procédé selon l'invention possède un répertoire antigénique similaire et/ou supérieur au répertoire antigénique des concentrés de l'art antérieur. En effet, les pertes limitées en immunoglobulines lors du procédé selon l'invention permettent avantageusement de garder une répartition des immunoglobulines similaire à celle du plasma et de conserver un panel antigénique large.

Le procédé selon l'invention est ainsi particulièrement avantageux pour la production de fractions d'immunoglobulines dirigées contre une cible antigénique spécifique.

De même, le procédé selon l'invention est particulièrement intéressant pour la préparation d'un concentré d'albumine, notamment à partir d'une fraction plasmatique déjà déplétée en immunoglobulines. L'étape de chromatographie multicolonnes selon l'invention permet un rendement de capture de plus de 90%. Alors que la capacité du gel lors d'une étape de chromatographie classique est généralement comprise entre 25 et 30 g/l de gel, l'utilisation d'une chromatographie multicolonnes échangeuse d'anions ou interactions hydrophobes ou une combinaison de ligands chimiques (multi-modes) selon l'invention permet d'obtenir une capacité de plus de 50 g/l de gel soit une saturation totale de la capacité dynamique du support.

Les exemples suivant illustrent l'invention, sans toutefois en limiter la portée.

### Exemple 1 : Préparation de concentrés d'immunoglobulines par chromatographie multicolonnes d'affinité à partir de cryo-surnageant

### Matériel & méthode

### Cryo-surnageant

Deux lots d'environ 5L de cryosurnageant de plasma humain (lots 13500-13L-06002 et13500-13L-06007) comprenant entre 8 et 10 g/L-1 d'IgG sont aliquotés en fractions de 700 mL puis congelés à -80°C. Les caractéristiques des lots utilisés sont reproduites dans le tableau 1 ci-dessous.

**Tableau 1 : Répartition des sous-classes d'IgG dans les cryosurnageants**

| **Matière première** | **N° de lot** | **IgG Totales (%)** | **IgG1 (%)** | **IgG2 (%)** | **IgG3 (%)** | **IgG4 (%)** |
|---|---|---|---|---|---|---|
| Cryo Surnageant | 13L06002 | 100,0 | 57,9 | 32,0 | 3,5 | 6,6 |
| Cryo Surnageant | 13L06007 | 100,0 | 57,2 | 32,3 | 4,0 | 6,6 |

Lors d'un essai, le volume nécessaire de cryosurnageant est décongelé pendant 20-30 min dans un bain marie à 37°C sans que le produit ne dépasse la température interne de 25°C.

### Solutions tampon

La composition des solutions tampon utilisées lors des différentes étapes du procédé de chromatographie d'affinité est résumée dans le tableau 2 ci-dessous.

**Tableau 2 : Solutions tampon pour la chromatographie d'affinité**

| Phases | Composition | Valeurs cibles |
|---|---|---|
| Equilibrage et Lavage | Na2HPO4, 12 H2O :16,7mM | pH=7,4 |
| | NaH2PO4, 2 H2O : 3,34 mM | |
| | NaCl 150 mM | |
| Elution | Glycine 0,1M | pH=3 |
| Ajustement Eluat | Glycine 0,1M | pH=11 |

La composition des solutions tampon utilisées lors des différentes étapes du procédé de chromatographie à échange d'ions est résumée dans le tableau 3 ci-dessous.

**Tableau 3 : Solutions tampon pour la chromatographie à échange d'ions.**

| Phases | Composition | Valeurs cibles |
|---|---|---|
| Pré-équilibrage | Glycine, NaCl | *pH: 9,0* +/- *0,1* |
| | | *conductivité: 8,5* +/- *0,5 mS*/*cm* |
| Equilibrage et Lavage | Glycine, NaCl | *pH: 9,0* +/- *0,1* |
| | | *conductivité: 1000* +/- *100 µS*/*cm* |
| Elution | Na2HPO4, 12H2O: | *pH: 6,2* +/- *0,1* |
| | NaH2PO4, 2H2O | *conductivité: 1600* +/- *100 µS*/*cm* |

### Gels de chromatographie

Pour la chromatographie d'affinité, on utilise un gel Ig Select® de GE Healthcare (lots n°10035817 et n° 10017479). Le ligand d'affinité utilisé est un ligand de la société BAC (BioAffinityCompany), qui fixe spécifiquement le fragment Fc des IgG humaines. Ce ligand est une protéine recombinante de 14kD, couplée à la base de la matrice par un long bras carboné d'espacement (spacer) qui facilite l'adsorption des Ig. Celui-ci est couplé au spacer par l'intermédiaire de liaisons amides multipoints.

Pour la chromatographie échangeuse d'ions, on utilise un gel échangeur d'anions fort Fractogel® EMD TMAE (lot n° 09L03583). Ce gel est constitué d'une résine polyméthacrylate réticulée sur laquelle sont greffés des groupes de trimethylaminoethyl (TMAE).

### Colonnes

Pour la chromatographie d'affinité multicolonnes, de 3 à 5 colonnes radiales de la société Proxcys, référencées µ-RFC 5.0-6.0 (lot 1303B014 - Volume de gel : 10mL ; hauteur de gel : 12 cm ; ratio diamètre d'entrée/diamètre de sortie : 2/1) ont été utilisées en combinaison avec un automate BioSc Lab (Novasep).

Pour la chromatographie TMAE, une colonne XK 16 (GE healthcare) volume de gel 41 mL de hauteur 20,4 cm, et surface 2,0 cm2 a été utilisée avec un automate AKTA Purifier 10 (GE healthcare).

### Ultrafiltration

On utilise une cassette Millipore Biomax 30 (PES) avec un cut-off de 30 kDa et une surface de 50 cm2 (référence C1PA45544).

### Filtration stérilisante

On utilise des filtres Sartorius Minisart de 5 cm2 de surface présentant une porosité de 0.45 µm et 0,2 µm (références 16537 et 16532).

### Essais

### Chromatographie d'affinité multicolonnes

Les essais ont été menés sur 4 colonnes, connectées de façon séquentielles en série lors des phases d'adsorption et de lavage du gel, avec une charge de 26 g d'IgG.L-1 de gel, un temps de contact de 5 minutes, à pH de fixation compris entre 7,3 et 7,8, l'élution étant réalisée avec une solution de glycine 0.1M pH3.

Le gel a été régénéré après chaque chromatographie.

La régénération a consisté à passer 2 VC de solution de chlorure de sodium 2M.

Le suivi de la chromatographie a été réalisé par enregistrement de la DO à 280 nm et par le calcul du rendement en IgG (dosage néphélémétrie).

Le débit de travail était de 2,3 mL.min⁻¹ ce qui correspond à un temps de contact de 2 min lors de l'adsorption.

Le déroulement des étapes de la chromatographie d'affinité multicolonnes est résumé dans le tableau 4 ci-dessous.

**Tableau 4 : Etapes de la chromatographie d'affinité multicolonnes**

| Etape | Solution | Volume | Remarques |
|---|---|---|---|
| Equilibrage | Tampon | Au minimum | Vérification du pH (7,4 ± 0,2) |
| | Equilibrage | 2VC | |
| Adsorption | Cryosurnageant | 3 mL à 22 mL selon essai | Collecte de la fraction non adsorbée par suivi de la DO 280 nm |
| Lavage | Tampon | 2 à 4 VC | Jusqu'au retour Ligne de base |
| | Equilibrage | | |
| Elution | Glycine 0,1M | 2 VC | Collecte jusqu'au retour à la ligne de base. |
| | pH 3,0 | | |
| Régénération | NaCl 2 M | 2 VC | / |
| Rééquilibrage de la colonne | Tampon | Au minimum | Vérification du pH (7,4 ± 0,2) |
| | Equilibrage | 2VC | |
| Stockage | Ethanol 20% | Au minimum | Non réalisée si réutilisation immédiate de la colonne |
| | | 2VC | |

| | | | |
|---|---|---|---|
| VC : volume colonne | | | |

### Traitement S/D et Chromatographie TMAE

Un Traitement S/D est effectué pendant environ 30 minutes pour inactiver les virus enveloppés. Le produit est ensuite ajusté en pH et en conductivité avant injection sur le gel TMAE.

La fraction non adsorbée du gel TMAE est ensuite récupérée (fraction éliminée), après retour à la ligne de base et lavage de la colonne, l'éluat du gel est récupéré pour la phase suivante.

### Ultrafiltration

L'étape d'ultrafiltration permet de dialyser et de concentrer l'éluat de la colonne TMAE à une concentration intermédiaire de 80-120.L-1.

### Formulation

Le produit a été formulé par addition du tampon de formulation (mannitol, glycine, Polysorbate 80). La concentration du produit finale est ajustée à 50g.L-1.

Le produit obtenu est filtré stérilement sur une séquence de filtres 0,45 et 0,22 µm. Il est ensuite échantillonné et conservé à l'état liquide à température de 4,0 - 7,0C.

### Résultats

Les analyses ont été réalisées sur le produit final afin d'être comparées à une Immunoglobuline IgG à 50 G/Litre, (produit de référence) obtenue suivant un procédé ayant en premières étapes de purification un fractionnement par l'éthanol
- Dosage des IgG totales et sous-classes par Néphélémétrie (tableau 6).
- Dosage des IgA, IgM et IgE par ELISA (Enzyme-Linked Immunosorbent Assay) (tableau 7).

**Tableau 5 : Répartition des sous-classes d'IgG**

| Essai | IgG totales (%) | IgG1 (%) | IgG2 (%) | IgG3 (%) | IgG4 (%) |
|---|---|---|---|---|---|
| Produit final (Charge IgSelect 26 g.L-1) | 100,0 | 53,4 | 33,4 | 1,2 | 1,7 |
| Produit de Référence | 100,0 | 59,7 | 36,8 | 2,4 | 1,9 |

**Tableau 6 : Analyse de la teneur en protéines contaminantes**

| Essai | Ig G | Ig A | Ig M | Ig E |
|---|---|---|---|---|
| | (g.L-1) | (mg.L-1) | (mg.L-1) | (UI.mL-1) |
| Produit final (Charge IgSelect 26 g.L-1) | 57,6 | < 13 | < 8 | 1,5 |
| Produit de Référence | 46,1 | 8,5 | <8 | <0,8 |

On constate que ces conditions de purification des IgG directement à partir de cryosurnageant donnent un produit de qualité équivalente au produit de référence. De plus, comme indiqué dans le tableau 6 ci-dessus, on observe une conservation de toutes les sous-classes d'IgG, avec une répartition se rapprochant de celle du plasma.

De façon plus intéressante, la chromatographie multicolonnes d'affinité utilisant le gel IgSelect a un rendement d'étape supérieur à 90%, et 7,4g d'IgG récupérés par litre de cryosurnageant. On constate une déplétion complète en IgG dans la fraction non adsorbée du gel d'affinité.

**Tableau 7 : Rendement affinité Ig Select Multicolonnes**

| Essai Charge IgSelect 26 g/L | Ig G (g.L-1) | Volume (L) | Ig G (mg) | Rendement Etape (%) / g IgG par litre de plasma |
|---|---|---|---|---|
| Cryosurnageant | 8,0 | 880 | 6500 | 100/8,0 |
| Eluat affinité Ig Select multicolonnes | 4,8 | 1245 | 6010 | 92,6/7 ,4 |

En conclusion, la purification des IgG directement à partir de cryosurnageant de plasma par chromatographie multicolonnes est tout à fait envisageable à l'échelle industrielle, notamment pour traiter des volumes de plusieurs milliers de litres de plasma par jour.

Par exemple, pour le traitement d'un lot industriel de 4500L de plasma humain, on peut utiliser 4 colonnes de 50L et de 12cm de hauteur de gel, avec une charge en cryosurnageant correspondant à 27g d'IgG par Litre de gel, un débit linéaire d'adsorption compris entre 100 et 300 cm.h-1, et un débit linéaire pour les étapes de lavage du gel et d'élution supérieur, compris entre 200 et 600 cm/Heure. Des configurations composées de 3 colonnes de 70 Litres de gel, ou de 5 colonnes de 40 Litres de gel sont envisageables, le nombre de cycles à réaliser pour traiter la totalité du lot étant déterminé en fonction du volume de matière première mise en oeuvre.

Une telle mise en oeuvre, même à l'échelle industrielle, permet d'obtenir un concentré d'immunoglobulines répondant aux requis règlementaires et possédant toutes les qualités requises à une utilisation pharmaceutique (pureté, sécurisation biologique, répartition des sous-classes d'IgG, etc.). Notamment, on observe une conservation des quatre sous-classes d'IgG du plasma. Afin d'éliminer le mélange solvant/détergent, les IgA et les IgE, une chromatographie TMAE peut ensuite être envisagée.

Par ailleurs, il est possible de prévoir une étape de lavage du gel avec du polysorbate 80%, soit simultanément à la pré-élution au NaCl, soit en mélange dans la fraction d'urée / acide acétique.

### Exemple 2 : Capture d'immunoglobulines par chromatographie multicolonnes d'affinité à partir de plasma

### Matériel & méthode

Des poches de plasma humain ayant permis de constituer un pool de plasma d'environ 30L comprenant entre 8 et 10 g/L-1 d'IgG ont été décongelées dans un bain marie à 37°C sans que le produit ne dépasse la température interne de 25°C afin d'extraire par une première chromatographie multicolonnes les immunoglobulines.

**Tableau 8 : Répartition des sous-classes d'IgG dans les pools de plasma**

| **Matière première** | **Essai N°** | **IgG Totales (%)** | **IgG1 (%)** | **IgG2 (%)** | **IgG3 (%)** | **IgG4 (%)** |
|---|---|---|---|---|---|---|
| Pool de plasma 1 | 1647-120 | 100,0 | 58,0 | 33,6 | 3,3 | 5,2 |
| Pool de plasma 2 | 1647-121B | 100,0 | 57,4 | 32,5 | 3,4 | 6,7 |

### Solutions tampon

La composition des solutions tampon utilisées lors des différentes étapes du procédé de chromatographie d'affinité est résumée dans le tableau 2 ci-dessous.

**Tableau 9 : Solutions tampon pour la chromatographie d'affinité**

| Phases | Composition | Valeurs cibles |
|---|---|---|
| Equilibrage et Lavage | Citrate de Sodium 10,0 mM, | pH=7,4 |
| | NaCl 100 mM | |
| Pré-élution | Citrate de Sodium 10 mM, | pH 7,4 |
| | NaCl 2,0 M | |
| Elution | Acide acétique | QSP pH=3,0 |
| Ajustement Eluat | Sodium Hydroxyde 1 M | QSP pH 4,8 |

### Gel de chromatographie

Pour la chromatographie d'affinité, on utilise un gel d'affinité CaptureSelect FcXL de la société Life Technologies (Ref. 19432801L, lot n° 200814-03). Le ligand d'affinité utilisé est un ligand de la société BAC (BioAffinityCompany), qui fixe spécifiquement le domaine CH3 des 4 sous-classes des IgG humaines. Ce ligand est une protéine recombinante de 14kD.

### Colonnes

Pour la chromatographie d'affinité multicolonnes, 4 colonnes radiales de la société Proxcys, (MD 122 MK III - Volume de gel : 250 mL par colonne pour un volume total de gel d'affinité de 1,0 L de gel; hauteur de gel : 12 cm ; ratio diamètre d'entrée/diamètre de sortie : 2/1) ont été utilisées en combinaison avec un automate Pilote BioSc M (Novasep).

### Essais

### Chromatographie d'affinité multicolonnes

Les essais ont été menés sur 4 colonnes, pilotées de façon séquentielles par l'automate avec une charge en plasma correspondant à 21 g d'IgG.L-1 de gel, un temps de contact de 3 à 4 minutes, à pH d'adsorption du plasma non modifié compris entre 7,1 et 7,8, l'élution étant réalisée avec une solution acide acétique à pH 3,0 Le gel a été régénéré après chaque chromatographie.

Le suivi de la chromatographie a été réalisé par enregistrement de la DO à 280 nm et par le calcul du rendement en IgG sur le pool des éluats (dosage par néphélémétrie). Le déroulement des étapes de la chromatographie d'affinité multicolonnes est résumé dans le tableau 10 ci-dessous.

**Tableau 10 : Etapes de la chromatographie d'affinité multicolonnes**

| Etape | Solution | Remarques |
|---|---|---|
| Equilibrage | Tampon | (7,4 ± 0,2) |
| | Equilibrage | |
| Adsorption | Plasma filtré 0,2 µm | Collecte de la fraction non adsorbée |
| Pré-élution | Citrate de Sodium 10 mM, NaCl 2 M | |
| Elution | Acide acétique pH 3,0 | Collecte jusqu'au retour à la ligne de base. |

Afin de limiter les risques d'agrégation des IgG à pH trop acide, les éluats des colonnes d'affinité obtenus ont été ajustés à pH 4,8 à l'aide d'une solution d'Hydroxyde de Sodium 1 M.

### Diafiltration concentration de l'éluat de chromatographie d'affinité FcXL multicolonnes.

L'éluat a été diafiltré en eau et concentré sur une membrane ayant un seuil de coupure de 30 kDa afin d'obtenir une concentration d'environ 70 g/L.

### Résultats

Les analyses ont été réalisées sur l'éluat de la chromatographie multicolonnes concentré par ultrafiltration.
- Dosage des IgG totales et sous-classes par Néphélémétrie (tableau 11).
- Dosage des IgA, IgM et IgE par ELISA (Enzyme-Linked Immunosorbent Assay) (tableau 12).

**Tableau 11 : Répartition des sous-classes d'IgG réalisé après ultrafiltration et concentration des éluats**

| Essai | IgG totales (%) | IgG1 (%) | IgG2 (%) | IgG3 (%) | IgG4 (%) |
|---|---|---|---|---|---|
| Eluat FcXL: plasma 1 | **100,0** | **58,3** | **32,9** | **3,0** | **5,7** |
| Eluat FcXL plasma 2 | **100,0** | **58,5** | **34,0** | **2,2** | **5,3** |

**Tableau 12 : Analyse de la teneur en protéines contaminantes réalisée après ultrafiltration et concentration des IgG**

| Essai | Ig G (g.L-1) | Ig A (g.L-1) | Ig M (g.L-1) | Ig E (UI.mL-1) |
|---|---|---|---|---|
| Eluat FcXL: plasma 1 | **76,6** | **1,00** | **0,75** | **33,0** |
| Eluat FcXL plasma 2 | **66,8** | **0,80** | **0,50** | **12,0** |

On constate que ces conditions de purification des IgG directement à partir de plasma donnent un produit de très bonne qualité. De plus, comme indiqué dans le tableau 11 ci-dessus, on observe une conservation de toutes les sous-classes d'IgG, avec une répartition se rapprochant de celle du plasma.

De façon plus intéressante, la chromatographie multicolonnes d'affinité utilisant le gel FcXL a un rendement d'étape supérieur à 90%, soit supérieur à 7,5 g d'IgG récupérés par litre de plasma mis en oeuvre. On constate une déplétion complète en IgG dans la fraction non adsorbée du gel d'affinité. En outre, l'albumine et le fibrinogène sont récupérés en totalité dans la fraction non adsorbée du gel.

**Tableau 13 : Rendement affinité FcXL Multicolonnes (stade éluat ajusté pH 4,8)**

| Essai | Ig G (g.L-1) | Volume (L) | Ig G (g) | Rendement Etape (%) / g IgG par litre de plasma |
|---|---|---|---|---|
| Plasma 1 | 4,4 | 60,7 | 264,0 | 92,1 / 7,5 |
| Plasma 2 | 3,2 | 70,4 | 225,3 | 97,9 / 7,9 |

En conclusion, la purification des IgG directement à partir du plasma par chromatographie multicolonnes est tout à fait envisageable à l'échelle industrielle, notamment pour traiter des volumes de plusieurs milliers de litres de plasma par jour.

**Tableau 14 : Comparaison de la capacité de capture des IgG avec le gel d'affinité FcXl en chromatographie classique et chromatographie multicolonnes (tampons de chromatographie équivalents).**

| **Type de chromatographie** | **Capacité en g IgG par litre de gel** |
|---|---|
| Classique | 15 |
| mulicolonnes | 21 |

Une telle mise en oeuvre, même à l'échelle industrielle, permet de capturer de façon extrêmement efficace les immunoglobulines présentes dans le plasma. Elle permet également d'obtenir, en une seule étape, un éluat de grande pureté qui conserve les qualités attendues d'un pool d'immunoglobulines thérapeutiques. Notamment, on observe une conservation des quatre sous-classes d'IgG du plasma et une bonne élimination des IgA, IgM et IgE. Afin d'éliminer les traces résiduelles d'IgA, IgM et IgE, une chromatographie d'échange d'ions de type TMAE (Merck) peut ensuite être envisagée.

### Exemple 3 : Capture d'albumine par chromatographie multicolonnes échangeuse d'ions, interactions hydrophobes ou une combinaison de ligands chimiques (multi-modes)

### Matériel & méthode

Du plasma déplété en IgG, récupéré à l'issu du procédé de purification des IgG selon l'exemple 2 est utilisé, l'albumine n'étant pas du tout fixée au gel d'affinité utilisé.

Les caractéristiques de la fraction plasmatique sont:
Albumine humaine 16.15 g/L, pH=6.5, conductivité de 12 mS/cm, en tampon citrate 0.01M
La composition des solutions tampon utilisées lors des différentes étapes du procédé de chromatographie est résumée dans le tableau 15 ci-dessous.

**Tableau 15 : Solutions tampon pour la chromatographie multicolonnes mixe-mode.**

| **Phase** | Tampon/produit |
|---|---|
| **Pré-équilibrage** | Citrate 0.1M, NaCl 0.1M, ajusté pH=6.5 |
| **Equilibrage** | Citrate 0.01M, NaCl 0.1M, ajusté pH=6.5 |
| **Elution** | Citrate 0.01M et NaCl 0.1M, pH=3.9 |

### Gels de chromatographie mixte-mode

Pour la chromatographie de l'albumine, on utilise un gel mixe-mode salt tolerant de type HEA Hypercel .

### Colonnes

5 colonnes radiales en série de 10 mL chacune ont été utilisées en combinaison avec un automate BioSc Lab (Novasep). Six cycles de 164 minutes chacun sont réalisés, soit 17 heures environ de fonctionnement continu.

Les conditions de chromatographie multicolonnes appliquées dans cet exemple ont permis de confirmer une capture quasi complète de l'albumine, très peu d'albumine restant dans la fraction non adsorbée. Le tableau ci-dessous résume les rendements dans chaque fraction collectée et la pureté minimale atteinte dans la fraction d'intérêt purifiée.

**Tableau 16 : Rendement en albumine et estimation de la pureté par électrophorèse SDS-PAGE**

| **Rendement fraction non adsorbée** | **Rendement éluat** | **Rendement régénération** | **Pureté dans l'éluat** |
|---|---|---|---|
| 3.4% | 87.7% | 8.9% | 80% |

Un gain de capacité d'au moins 70% est observé au cours de l'essai en chromatographie multicolonnes comparativement à un essai classique où la charge est arrêtée lorsque 10% de fuite est atteint en sortie de colonne. Le tableau 17 résume le gain calculé pour les 2 conditions.

**Tableau 17 : Comparaison de la capacité de capture du gel HEA en chromatographie classique et chromatographie multicolonnes (tampons de chromatographie équivalents).**

| **Type de chromatographie** | **Capacité en g albumine par litre de gel** |
|---|---|
| Classique | 30 |
| Multicolonnes | 52 |

Les résultats du tableau 17 montrent que seulement 3,4% environ de l'albumine n'a pas été adsorbé. On observe une pureté électrophorétique de 80% environ.

Après l'élution à pH=3.9 un rendement de 88% a été obtenu. Cet essai montre qu'au moins 96% de l'albumine du plasma sanguin a été capturé par chromatographie selon un mode chromatographie multicolonnes sur le support HEA-HyperCel et au moins 88% de cette albumine a été récupérée à l'élution, ce qui correspond à un bilan capture/élution d'au moins 80%. La chromatographie multicolonnes sur ce gel a permis de faire passer la capacité du gel de 30 g/L en chromatographie classique à 52 g/L soit une réduction du volume de gel total nécessaire par lot de 70%.

Le plasma humain étant composé d'environ 40 g/L d'albumine, au moins 33 g/L peuvent être purifié selon cet exemple.

### Exemple 4 : Capture de fibrinogène par chromatographie multicolonnes d'affinité Matériel & méthode

### Fraction plasmatique

Du plasma déplété en IgG et en albumine, récupéré à l'issu du procédé de purification de l'albumine (passage pendant 14 heures sur les deux chromatographies multicolonnes) selon l'exemple 3 est utilisé après avoir été congelé et stocké à -80°C puis décongelé le jour de la capture du fibrinogène par chromatographie.

Caractéristiques de la fraction plasmatique : Fibrinogène Antigénique : 0,32 g/L.

### Solutions tampon

La composition des solutions tampon utilisées lors des différentes étapes du procédé de chromatographie est résumée dans le tableau 18 ci-dessous.

**Tableau 18 : Solutions tampon pour la chromatographie d'affinité.**

| Phases | Composition | Valeurs cibles |
|---|---|---|
| Equilibration de la colonne et retour à la ligne de base | Citrate tri-sodique 10 mM, | pH ajusté à 7,4 |
| | Chlorure de Sodium 100 mM | |
| Pré-élution | Chlorure de Sodium 2,0 M | pH ajusté à 7,0 |
| Elution | Trométamol (Tris HCl) 20 mM, | |
| | Chlorure de Magnésium 1,5 M, | pH ajusté à 7,0 |
| | Propylène Glycol 20 % v/v, | |
| | Arginine 200 mM | |

### Gels de chromatographie d'affinité

Pour la chromatographie, on utilise un gel d'affinité CaptureSelect Fibrinogen (Life Technologies ref. 191291050, lot 171013-01) dont les caractéristiques de capture du fibrinogène sont résumées dans le tableau 19 ci-dessous..

**Tableau 19 : Capacité du gel CaptureSelect Fibrinogen**

| | |
|---|---|
| **Charge en fibrinogène** | 10 g/L de gel |

### Colonnes

Une colonne axiale de 5 mL (Référence Tricorn 5/100 (GE Healthcare), volume colonne : 1,6 ml ; hauteur de colonne :8 cm;) a été utilisée

### Purification par affinité

Le plasma appauvri en Immunoglobulines et albumine décongelé est injecté sans modification sur la colonne d'affinité CaptureSelect Fibrinogène équilibrée. Une charge d'environ 10 g/L a été appliquée. La teneur de l'éluat en fibrinogène a été dosé par méthode ELISA.

### Résultats

A la fin du procédé, le rendement en fibrinogène est de 70 %. Le produit obtenu a une concentration en fibrinogène antigénique de 1,3 mg/ml.

Cet essai démontre clairement qu'il est possible de capturer les trois protéines abondantes du plasma en n'utilisant successivement que des étapes de chromatographie (en dehors d'étapes d'inactivation virales et de filtration).

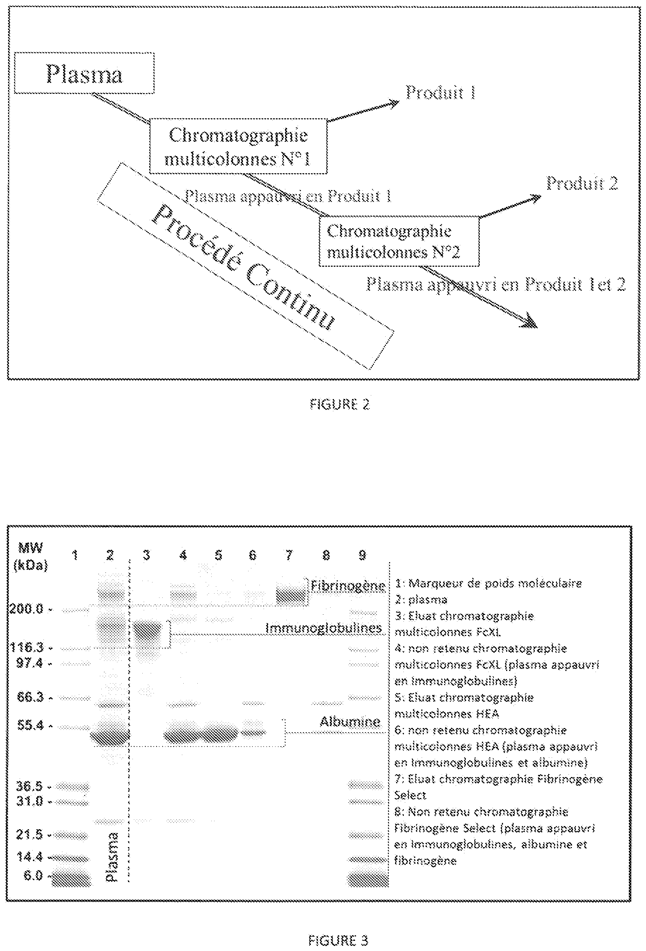

Dans cet exemple deux chromatographies multicolonnes en continu et une chromatographie classique sont réalisées à la suite les unes des autres, en retraitant successivement les fractions non adsorbées. Cet enchainement de chromatographies (Figure 2) sur plus de 10 heures n'est pas préjudiciable à la qualité des molécules capturées et tout particulièrement la molécule ayant été purifiée en dernier, dans le cas présent le fibrinogène. Celui-ci a conservé très majoritairement son intégrité tout au long du procédé de purification, comme le montre le SDS-PAGE en conditions non réductrices et coloration bleu de Coomassie des différentes chromatographies successives de capture des immunoglobulines, suivie de la capture de l'albumine et enfin suivie de la capture du fibrinogène (figure 3).

Sur cette figure 3, le plasma de départ puis les éluats et les fractions non-retenues montrent l'appauvrissement successif du plasma vis-à-vis des molécules capturées et l'enrichissement des éluats en molécule capturées.

Le puits 1 correspond aux marqueurs de poids moléculaire et le puits 2 correspond au plasma normal non purifié.

Les puits 3 et 4 correspondent aux fractions obtenues à l'exemple 2.

Les puits 5 et 6 correspondent aux fractions obtenues à l'exemple 3.

Les puits 7 et 8 correspondent aux fractions obtenues à l'exemple 4.

Le fibrinogène d'un poids moléculaire de 340 kDa conserve majoritairement son intégrité moléculaire dans le non retenu de la chromatographie multicolonnes de capture des immunoglobulines puis dans le non retenu de la chromatographie multicolonnes de capture de l'albumine. On observe dans le non retenu de la chromatographie d'affinité de capture du fibrinogène l'absence de fibrinogène qui est totalement capturé et se retrouve avec une structure majoritairement intègre dans l'éluat de cette chromatographie.

## Revendications

1. Procédé de préparation de concentrés de protéines plasmatiques purifiées à usage thérapeutique à partir de plasma sanguin comprenant des étapes de purification selon lesquelles on soumet successivement du plasma sanguin ou une fraction plasmatique à trois chromatographies multicolonnes, de manière à récupérer successivement trois concentrés de protéines plasmatiques purifiées, lesdits concentrés de protéines plasmatiques purifiées à usage thérapeutique étant un concentré d'immunoglobulines, un concentré de fibrinogène et un concentré d'albumine.

2. Procédé selon la revendication 1, dans lequel au moins une chromatographie multicolonnes est une chromatographie multicolonnes d'affinité.

3. Procédé selon l'une quelconque des revendications précédentes, dans lequel on soumet directement du plasma ou du surnageant de plasma cryoprécipité à une première étape de purification des immunoglobulines humaines consistant en une chromatographie multicolonnes d'affinité.

4. Procédé selon l'une des revendications 2 ou 3, dans lequel la chromatographie multicolonnes d'affinité met en oeuvre un ligand d'affinité choisi parmi les anticorps, les fragments d'anticorps, les dérivés d'anticorps, les ligands chimiques tels que des peptides, des peptides mimétiques, des peptoïdes, des nanofitines, et les ligands oligonucléotidiques tels que les aptamères, le ligand d'affinité étant optionnellement choisi parmi les ligands d'affinité résistants aux conditions de sanitisation et/ou de réutilisation intensive compatibles avec une utilisation industrielle.

5. Procédé selon l'une quelconque des revendications précédentes, dans lequel au moins une chromatographie multicolonnes est une chromatographie multicolonnes échangeuse d'ions, telle qu'une chromatographie multicolonnes à échange d'anions ou de cations, ou une chromatographie multicolonnes hydrophobe, ou une chromatographie multicolonnes en mode mixte ou une chromatographie multicolonnes à exclusion de taille.

6. Procédé selon la revendication 5, dans lequel la chromatographie multicolonnes à échange d'anions est mise en oeuvre sur un gel de polysaccharide réticulé ou de polymère vinylique ou acrylique, greffé de groupements DEAE ou TMAE ou QAE.

7. Procédé selon la revendication 6, dans lequel la chromatographie multicolonnes à échange d'anions et/ou en mode mixte est réalisée sur une matrice à haute tolérance au sel pour capturer la protéine plasmatique dans un milieu ayant une salinité élevée tel que le plasma ou la fraction non retenue d'une chromatographie.

8. Procédé selon l'une des revendications 1 à 7, dans lequel la première chromatographie multicolonnes est une chromatographie multicolonnes échangeuse d'ion selon laquelle on soumet directement du plasma ou du surnageant de plasma cryoprécipité à une chromatographie multicolonnes à échange d'anions, et optionnellement dans lequel on soumet la fraction contenant lesdites immunoglobulines humaines à une étape ultérieure de purification par précipitation à l'acide caprylique et on récupère le surnageant contenant les immunoglobulines.

9. Procédé selon l'une des revendications 1 à 8, dans lequel la première chromatographie multicolonnes est une chromatographie multicolonnes d'affinité selon laquelle on soumet directement du plasma ou du surnageant de plasma cryoprécipité à une étape de purification de l'albumine et/ou du fibrinogène, et optionnellement dans lequel on soumet la fraction contenant l'albumine et/ou le fibrinogène à une étape ultérieure de purification par précipitation à l'acide caprylique et on récupère la fraction contenant l'albumine et/ou le fibrinogène.

10. Procédé selon l'une des revendications précédentes, dans lequel la fraction retenue d'au moins une chromatographie multicolonnes d'affinité ou échangeuse d'ions est la protéine plasmatique d'intérêt, ladite protéine plasmatique d'intérêt étant ensuite éluée.

11. Procédé selon l'une des revendications précédentes, dans lequel la fraction retenue d'au moins une chromatographie multicolonnes d'affinité ou échangeuse d'ions contient les contaminants, la fraction non retenue comprenant la protéine d'intérêt.

12. Procédé de fractionnement de plasma sanguin comprenant les étapes consistant à soumettre directement du plasma sanguin ou du surnageant de plasma cryoprécipité ou une fraction plasmatique à :
- une chromatographie multicolonnes d'affinité dans laquelle au moins un ligand d'affinité est un ligand liant spécifiquement les immunoglobulines, à l'issue de laquelle on récupère la fraction contenant les immunoglobulines; et
- une chromatographie multicolonnes échangeuse d'ions et/ou mode mixte, optionnellement hautement tolérante au sel, à l'issue de laquelle on récupère la fraction contenant le l'albumine et/ou le fibrinogène; et
- une étape de purification de ladite fraction non retenue de la chromatographie multicolonne précédente contenant le fibrinogène et/ou l'albumine par précipitation et/ou par chromatographie.

13. Procédé de fractionnement de plasma sanguin comprenant les étapes consistant à soumettre directement du plasma sanguin ou du surnageant de plasma cryoprécipité ou une fraction plasmatique à :
- une chromatographie multicolonnes d'affinité dans laquelle au moins un ligand d'affinité est un ligand liant spécifiquement les immunoglobulines, à l'issue de laquelle on récupère la fraction contenant les immunoglobulines; et
- une chromatographie multicolonnes d'affinité dans laquelle au moins un ligand d'affinité est un ligand liant spécifiquement le fibrinogène, à l'issue de laquelle on récupère la fraction contenant le fibrinogène ; et
- une chromatographie multicolonnes d'affinité dans laquelle au moins un ligand d'affinité est un ligand liant spécifiquement l'albumine, à l'issue de laquelle on récupère la fraction contenant l'albumine.

## Patentansprüche

1. Verfahren zur Herstellung gereinigter Plasmaproteinkonzentrate aus Blutplasma für eine therapeutische Verwendung, umfassend Reinigungsschritte, wobei das Blutplasma oder eine Plasmafraktion nacheinander drei Mehrsäulen-Chromatographien unterzogen wird, um nacheinander drei gereinigte Plasmaproteinkonzentrate wiederzugewinnen, wobei besagte gereinigte Plasmaproteinkonzentrate zur therapeutischen Verwendung ein Immunglobulinkonzentrat, ein Fibrinogenkonzentrat und ein Albuminkonzentrat sind.

2. Verfahren gemäß Anspruch 1, wobei wenigstens eine Mehrsäulen-Chromatographie eine Mehrsäulen-Affinitätschromatographie ist.

3. Verfahren gemäß einem der vorhergehenden Ansprüche, wobei das Plasma oder der Überstand eines kryopräzipitierten Plasmas direkt einem ersten Reinigungsschritt von humanen Immunglobulinen unterzogen wird, der aus einer Mehrsäulen-Affinitätschromatographie besteht.

4. Verfahren gemäß einem der Ansprüche 2 oder 3, wobei die Mehrsäulen-Affinitätschromatographie einen Affinitätsligand verwendet, ausgewählt aus Antikörpern, Antikörperfragmenten, Antikörperderivaten, chemischen Liganden, wie Peptiden, mimetischen Peptiden, Peptoiden, Nanofitinen, und Oligonukleotid-Liganden wie Aptameren, wobei der Affinitätsligand gegebenenfalls aus Affinitätsliganden ausgewählt ist, die den Sanitisierungsbedingungen und/oder einer mit einer industriellen Nutzung verträglichen intensiven Wiederverwendung standhalten.

5. Verfahren gemäß einem der vorhergehenden Ansprüche, wobei wenigstens eine Mehrsäulen-Chromatographie eine Mehrsäulen-Ionenaustausch-Chromatographie, wie eine Mehrsäulen-Anionen- oder Kationenaustausch-Chromatographie, oder eine hydrophobe Mehrsäulen-Chromatographie oder eine gemischte Mehrsäulen-Chromatographie oder eine Mehrsäulen-Größenausschluss-Chromatographie ist.

6. Verfahren gemäß Anspruch 5, wobei die Mehrsäulen-Anionenaustausch-Chromatographie auf einem vernetzten Polysaccharidgel oder einem Vinyl- oder Acrylpolymergel durchgeführt wird, das mit DEAE oder TMAE oder QAE Gruppen gepfropft ist.

7. Verfahren gemäß Anspruch 6, wobei die Mehrsäulen-Anionenaustausch-Chromatographie und/oder die gemischte Mehrsäulen-Chromatographie auf einer Matrix mit einer hohen Salztoleranz erfolgt, um das Plasmaprotein in einem Medium mit einem hohen Salzgehalt, wie dem Plasma oder der von einer Chromatographie nicht-zurückgehaltenen Fraktion, einzufangen.

8. Verfahren gemäß einem der Ansprüche 1 bis 7, wobei die erste Mehrsäulen-Chromatographie eine Mehrsäulen-Ionenaustausch-Chromatographie ist, wobei das Plasma oder der Überstand eines kryopräzipitierten Plasmas direkt einer Mehrsäulen-Anionenaustausch-Chromatographie unterzogen wird und gegebenenfalls wobei die Fraktion, die besagte humane Immunglobuline enthält, einem weiteren Reinigungsschritt mittels Caprylsäure-Präzipitation unterzogen wird und der Überstand gesammelt wird, der die Immunglobuline enthält.

9. Verfahren gemäß einem der Ansprüche 1 bis 8, wobei die erste Mehrsäulen-Chromatographie eine Mehrsäulen-Affinitätschromatographie ist, wobei das Plasma oder der Überstand eines kryopräzipitierten Plasmas direkt einem Schritt der Reinigung von Albumin und/oder Fibrinogen unterzogen wird und gegebenenfalls wobei die Fraktion, die Albumin und/oder Fibrinogen enthält, einem weiteren Reinigungsschritt mittels Caprylsäure-Präzipitation unterzogen wird und die Fraktion gesammelt wird, die Albumin und/oder Fibrinogen enthält.

10. Verfahren gemäß einem der vorhergehenden Ansprüche, wobei die Fraktion, die von wenigstens einer Mehrsäulen-Affinitätschromatographie oder Mehrsäulen-Ionenaustausch-Chromatographie zurückgehalten wird, das Plasmaprotein von Interesse ist, wobei besagtes Plasmaprotein von Interesse anschließend eluiert wird.

11. Verfahren gemäß einem der vorhergehenden Ansprüche, wobei die Fraktion, die von wenigstens einer Mehrsäulen-Affinitätschromatographie oder Mehrsäulen-Ionenaustausch-Chromatographie zurückgehalten wird, die Kontaminanten enthält, wobei die nichtzurückgehaltene Fraktion das Protein von Interesse enthält.

12. Verfahren zur Fraktionierung von Blutplasma, umfassend die Schritte, bestehend aus direktem Unterziehen des Blutplasmas oder des Überstands eines kryopräzipitierten Plasmas oder einer Plasmafraktion:
- einer Mehrsäulen-Affinitätschromatographie, wobei wenigstens ein Affinitätsligand ein Ligand ist, der spezifisch die Immunglobuline bindet, an deren Ende die Fraktion gesammelt wird, die die Immunglobuline enthält; und
- einer Mehrsäulen-Ionenaustausch-Chromatographie und/oder gemischten Mehrsäulen-Chromatographie, gegebenenfalls hoch salztolerant, an deren Ende die Fraktion gesammelt wird, die Albumin und/oder Fibrinogen enthält; und
- einem Schritt der Reinigung besagter Fraktion, die von der vorhergehenden Mehrsäulen-Chromatographie nicht-zurückgehalten wird und Fibrinogen und/oder Albumin enthält, mittels Präzipitation und/oder mittels Chromatographie.

13. Verfahren zur Fraktionierung von Blutplasma, umfassend die Schritte, bestehend aus Unterziehen des Blutplasmas oder des Überstands eines kryopräzipitierten Plasmas oder einer Plasmafraktion:
- einer Mehrsäulen-Affinitätschromatographie, wobei wenigstens ein Affinitätsligand ein Ligand ist, der spezifisch die Immunglobuline bindet, an deren Ende die Fraktion gesammelt wird, die die Immunglobuline enthält; und
- einer Mehrsäulen-Affinitätschromatographie, wobei wenigstens ein Affinitätsligand ein Ligand ist, der spezifisch Fibrinogen bindet, an deren Ende die Fraktion gesammelt wird, die Fibrinogen enthält; und
- einer Mehrsäulen-Affinitätschromatographie, wobei wenigstens ein Affinitätsligand ein Ligand ist, der spezifisch Albumin bindet, an deren Ende die Fraktion gesammelt wird, die Albumin enthält.

## Claims

1. Process for preparing purified plasma protein concentrates for therapeutic use from blood plasma, comprising purification steps wherein blood plasma or a plasma fraction is successively subjected to three multicolumn chromatographies, so as to recover successively three purified plasma protein concentrates, said purified plasma protein concentrates for therapeutic use being an immunoglobulin concentrate, a fibrinogen concentrate and an albumin concentrate.

2. Process according to claim 1, wherein at least one multicolumn chromatography is multicolumn affinity chromatography.

3. Process according to any one of the preceding claims, wherein plasma or cryoprecipitated plasma supernatant is directly subjected to a first step of purification of human immunoglobulins consisting of multicolumn affinity chromatography.

4. Process according to claim 2 or claim 3, wherein the multicolumn affinity chromatography uses an affinity ligand selected from antibodies, antibody fragments, antibody derivatives, chemical ligands such as peptides, mimetic peptides, peptoids, nanofitins, and oligonucleotide ligands such as aptamers, the affinity ligand being optionally selected from affinity ligands resistant to conditions of sanitization and/or intensive reuse compatible with industrial use.

5. Process according to any one of the preceding claims, wherein at least one multicolumn chromatography is multicolumn ion-exchange chromatography, such as multicolumn anion- or cation-exchange chromatography, or multicolumn hydrophobic interaction chromatography, or multicolumn mixed-mode chromatography or multicolumn size-exclusion chromatography.

6. Process according to claim 5, wherein the multicolumn anion-exchange chromatography is performed on a crosslinked polysaccharide gel or a vinyl or acrylic polymer gel, grafted with DEAE or TMAE or QAE groups.

7. Process according to claim 6, wherein the multicolumn anion-exchange and/or mixed-mode chromatography is performed on a highly salt tolerant matrix in order to capture the plasma protein in a medium having high salinity such as the plasma or the fraction not retained by chromatography.

8. Process according to any one of claims 1 to 7, wherein the first multicolumn chromatography is multicolumn ion-exchange chromatography, wherein plasma or cryoprecipitated plasma supernatant is directly subjected to multicolumn ion-exchange chromatography, and optionally wherein the fraction containing said human immunoglobulins is subjected to a subsequent purification step by precipitation with caprylic acid and the supernatant containing immunoglobulins is collected.

9. Process according to any one of claims 1 to 8, wherein the first multicolumn chromatography is multicolumn affinity chromatography wherein plasma or cryoprecipitated plasma supernatant is directly subjected to a step of purification of albumin and/or fibrinogen, and optionally wherein the fraction containing albumin and/or fibrinogen is subjected to a subsequent purification step by precipitation with caprylic acid and the fraction containing albumin and/or fibrinogen is collected.

10. Process according to any one of the preceding claims, wherein the retained fraction from at least one multicolumn affinity or ion-exchange chromatography is the plasma protein of interest, said plasma protein of interest then being eluted.

11. Process according to any one of the preceding claims, wherein the retained fraction from at least one multicolumn affinity or ion-exchange chromatography contains the contaminants, the non-retained fraction comprising the protein of interest.

12. Process for fractionating blood plasma, comprising the steps consisting in directly subjecting blood plasma or cryoprecipitated plasma supernatant or a plasma fraction to:
- multicolumn affinity chromatography wherein at least one affinity ligand is a ligand specifically binding immunoglobulins, at the conclusion of which the fraction containing immunoglobulins is collected; and
- multicolumn ion-exchange and/or mixed-mode chromatography, optionally highly salt tolerant, at the conclusion of which the fraction containing albumin and/or fibrinogen is collected; and
- a step of purification of said fraction not retained by the preceding multicolumn chromatography containing fibrinogen and/or albumin by precipitation and/or chromatography.

13. Process for fractionating blood plasma, comprising the steps consisting in directly subjecting blood plasma or cryoprecipitated plasma supernatant or a plasma fraction to:
- multicolumn affinity chromatography wherein at least one affinity ligand is a ligand specifically binding immunoglobulins, at the conclusion of which the fraction containing immunoglobulins is collected; and
- multicolumn affinity chromatography wherein at least one affinity ligand is a ligand specifically binding fibrinogen, at the conclusion of which the fraction containing fibrinogen is collected; and
- multicolumn affinity chromatography wherein at least one affinity ligand is a ligand specifically binding albumin, at the conclusion of which the fraction containing albumin is collected.
